(19) Europäisches Patentamt
European Patent Office
Office européen des brevets

(11) Veröffentlichungsnummer: **0 111 448**
**B1**

(12) # EUROPÄISCHE PATENTSCHRIFT

(45) Veröffentlichungstag der Patentschrift: **13.01.88**

(51) Int. Cl.[4]: **G 03 C 7/26,** C 07 D 211/58, C 07 D 211/46, C 07 D 211/14, C 07 D 409/12, C 07 D 413/04

(21) Anmeldenummer: **83810560.9**

(22) Anmeldetag: **02.12.83**

(54) **Farbphotographisches Aufzeichnungsmaterial.**

(30) Priorität: **06.12.82 CH 7142/82**

(43) Veröffentlichungstag der Anmeldung:
**20.06.84 Patentblatt 84/25**

(45) Bekanntmachung des Hinweises auf die Patenterteilung:
**13.01.88 Patentblatt 88/2**

(84) Benannte Vertragsstaaten:
**BE DE FR GB IT**

(56) Entgegenhaltungen:
**EP-A-0 011 051**
**EP-A-0 013 443**
**EP-A-0 047 605**

**Die Akte enthält technische Angaben, die nach dem Eingang der Anmeldung eingereicht wurden und die nicht in dieser Patentschrift enthalten sind.**

(73) Patentinhaber: **CIBA- GEIGY AG, Klybeckstrasse 141, CH- 4002 Basel (CH)**

(72) Erfinder: **Leppard, David G., Route de Bourguillon 6, CH- 1723 Marly (CH)**
Erfinder: **Rody, Jean, Dr., Rütiring 82, CH- 4125 Riehen (CH)**

**EP 0 111 448 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Die vorliegende Anmeldung betrifft ein farbphotographisches Aufzeichnungsmaterial, das in mindestens einer lichtempfindlichen Silberhalogenidemulsionsschicht und/oder in mindestens einer der üblichen Hilfsschichten als Stabilisator eine spezifische Polyalkylpiperidinverbindung enthält.

Polyalkylpiperidine sind als sterisch gehinderte Amine allgemein als Lichtschutzmittel für organische Materialien, insbesondere für Polymere, bekannt. Es wurde auch bereits in der DE-OS 2 126 954 vorgeschlagen, solche Polyalkylpiperidine als Mittel gegen das Ausbleichen von Farbphotographien zu verwenden. Es wurde weiterhin in der EP-A 11051 vorgeschlagen, als Lichtschutzmittel für Farbphotographien bestimmte Polyalkylpiperidinderivate zu verwenden, die mindestens eine Phenolgruppe enthalten. Es handelt sich dabei um Polyalkylpiperidinester von Hydroxylbenzylmalonsäuren.

Es wurde nun gefunden, dass Polyalkylpiperidinverbindungen, die über eine Carboxy- oder Carbamingruppe verknüpftes sterisch gehindertes Phenol enthalten, wobei der Polyalkylpiperidinrest nicht direkt an die Carboxy- oder Carbamingruppe gebunden ist, eine überraschend bessere stabilisierende Wirkung entfalten.

Gegenstand der vorliegenden Erfindung ist daher ein farbphotographisches Aufzeichnungsmaterial, das in mindestens einer lichtempfindlichen Silberhalogenidemulsionsschicht, einer Zwischenschicht und/oder einer Schutzschicht mindestens eine Polyalkylpiperidinverbindung als Stabilisator enthält, dadurch gekennzeichnet, dass die Polyalkylpiperidinverbindung der Formel I

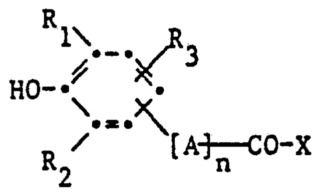

(I)

entspricht, worin

n die Zahlen 0 oder 1 bedeutet,

$R_1$ Wasserstoff, $C_1$-$C_8$ Alkyl, $C_5$-$C_8$ Cycloalkyl, Phenyl $C_7$-$C_{14}$ Aralkyl, $C_7$-$C_{14}$ Alkaryl oder eine Gruppe

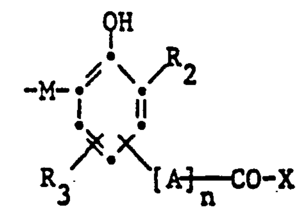

$R_2$ $C_1$-$C_8$ Alkyl, $C_5$-$C_8$ Cycloalkyl, Phenyl, $C_7$-$C_{14}$ Aralkyl, $C_7$-$C_{14}$ Alkaryl und

$R_3$ Wasserstoff oder Methyl sind,

M eine direkte Bindung -S-, -S-S-, -S(O)-, $-S(O)_2$ - oder $-CH(R_4)-$, worin $R_4$ Wasserstoff oder $C_1$-$C_8$ Alkyl ist, bedeutet,

A eine Gruppe $-CH_2-$, $-CH_2-CH(R_5)-$,

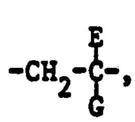, 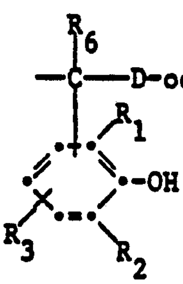 oder 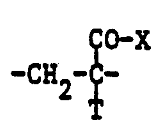

ist, worin

$R_5$ Wasserstoff, Methyl, Ethyl, Phenoxymethyl oder Phenyl ist,

$R_6$ Wasserstoff, $C_1$-$C_{12}$ Alkyl, $C_5$-$C_8$ Cycloalkyl, Phenyl oder Benzyl ist,

E -CN oder eine Gruppe $-COOR_7$, $-COR_8$, $-SO_2R_8$, $-P(O)(OR_9)_2$, -CHO, wobei $R_7$ $C_1$-$C_4$ Alkyl, $R_8$ $C_1$-$C_{12}$ Alkyl, $C_7$-$C_{14}$ Alkaryl, Phenyl und $R_9$ $C_1$-$C_{18}$ Alkyl, Phenyl oder Allyl sind,

G Wasserstoff, $C_1$-$C_{18}$ Alkyl, $C_3$-$C_{12}$ Alkenyl, $C_3$-$C_4$ Alkinyl, $C_3$-$C_{12}$ Cycloalkyl, $C_6$-$C_{18}$ Alkylcycloalkyl, $C_6$-$C_{14}$ Cycloalkylalkyl, $C_7$-$C_{14}$ Aralkyl, $C_7$-$C_{14}$ Alkaryl, Phenyl, durch Phenoxy, $C_7$-$C_{10}$ Alkylphenoxy, Benzyloxy, Cyclohexyloxy, Cyano, $-COOR_{10}$, $-OCOR_{11}$ oder $-P(O)(OR_{12})_2$ substituiertes $C_1$-$C_{18}$ Alkyl ist, wobei $R_{10}$ $C_1$-$C_{18}$ Alkyl, $C_3$-$C_{12}$ Cycloalkyl oder eine Gruppe der Formel II
$-(-C_aH_{2a}-)-CO-X$ (II),
worin a die Zahlen 1 bis 6 bedeutet,

$R_{11}$ $C_1$-$C_{18}$ Alkyl, $C_3$-$C_{12}$ Cycloalkyl, Phenyl, $C_7$-$C_9$ Phenylalkyl oder eine Gruppe der Formel III

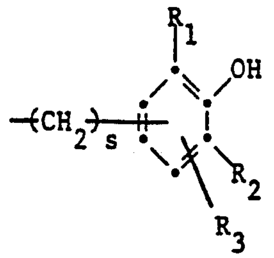

(III)

worin

s die Zahlen 0, 1 oder 2 bedeutet und

$R_{12}$ $C_1$-$C_8$ Alkyl, Allyl oder Phenyl sind, oder

G durch -O-, -S-, -SO-, $-SO_2-$ unterbrochenes $C_2$-$C_{18}$ Alkyl oder eine Gruppe der Formel III ist, oder wenn E eine Gruppe $-COR_8$ ist, G und $R_8$ zusammen unsubstituiertes oder durch Hydroxy oder Oxo substituiertes Tri- oder Tetramethylen bilden,

D eine Gruppe $-(C_rH_{2r}-)$ oder

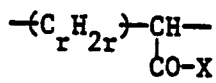

ist, worin

r die Zahlen 0 bis 12 bedeutet,

T Wasserstoff, $C_1$-$C_{18}$ Alkyl, $C_3$-$C_{12}$ Alkenyl, $C_3$-$C_4$ Alkinyl, $C_3$-$C_{12}$ Cycloalkyl, $C_6$-$C_{18}$ Alkylcycloalkyl, $C_6$-$C_{14}$ Cycloalkylalkyl, $C_7$-$C_{14}$ Aralkyl, $C_7$ -$C_{14}$ Alkaryl, Phenyl, Cyano oder eine Gruppe $-COR_8$, $-SO_2R_8$ oder $-P(O)(OR_9)_2$, eine Gruppe der Formel III, eine Gruppe der Formel IV

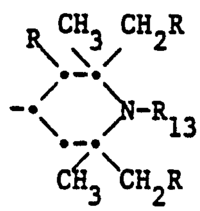

(IV)

oder eine Gruppe der Formel

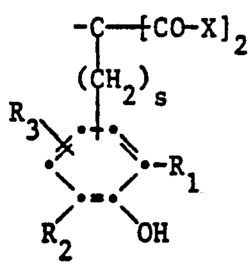

oder T durch -O-, -S-, -SO- oder $-SO_2-$ unterbrochenes $C_1$ -$C_{18}$ Alkyl ist,

X eine Gruppe der Formel

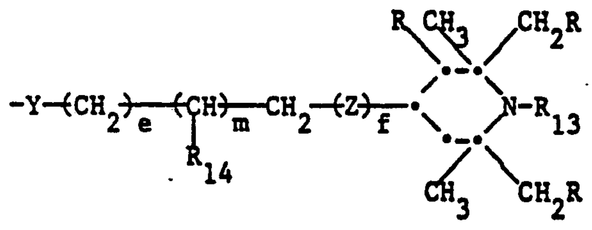

oder der Formel

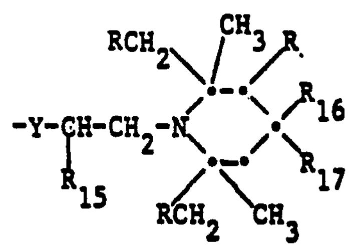

ist, worin
e die Zahlen 0 bis 5 und
f und m unabhängig voneinander die Zahlen 0 oder 1 bedeuten

R Wasserstoff oder Methyl ist

$R_{13}$ Hydroxy, $C_1$ -$C_{12}$ Alkyl, $C_3$ -$C_6$ Alkenylmethyl, $C_3$ -$C_4$ Alkinylmethyl, $C_7$ -$C_{14}$ Aralkyl, Glycidyl, durch Halogen, Cyano, -$COOR_{18}$ oder -$CON(R_{19})(R_{20})$ substituerters $C_1$ -$C_4$ Alkyl, eine Gruppe -$COR_{21}$, -$COOR_{18}$ oder -$CON(R_{19})(R_{20})$, eine Gruppe -$CH_2$-$CH(R_{22})$-$OR_{23}$, -$SOR_{24}$, -$SO_2R_{24}$, -$OR_{18}$, -$OCOR_{21}$ ist, wobei $R_{18}$ $C_1$-$C_{12}$ Alkyl, Allyl, Cyclohexyl oder Benzyl, $R_{19}C_1$-$C_{12}$ Alkyl, Allyl, Cyclohexyl, Benzyl, Phenyl oder $C_7$-$C_{10}$ Alkylphenyl, $R_{20}$ Wasserstoff, $C_1$-$C_{12}$ Alkyl, Allyl, Cyclohexyl oder Benzyl sind oder $R_{19}$ und $R_{20}$ zusammen mit dem N-Atom an das sie gebunden sind einen 5- oder 6-gliedrigen heterozyklischen Ring bilden, $R_{21}$ Wasserstoff, $C_1$-$C_{12}$ Alkyl, $C_2$-$C_6$ Alkenyl, Chloromethyl, $C_5$-$C_8$ Cycloalkyl, $C_7$-$C_{14}$ Aralkyl, Phenyl, $C_7$-$C_{10}$ Alkylphenyl oder durch 1 oder 2 $C_1$-$C_4$ Alkyl und 1 Hydroxy substituiertes Phenyl, Phenylmethyl oder Phenylethyl, $R_{22}$ Wasserstoff, $C_1$-$C_4$ Alkyl, $C_3$-$C_4$ Alkoxyalkyl, Phenyl oder Phenoxymethyl, $R_{23}$ Wasserstoff, $C_1$-$C_{12}$ Alkyl, eine Gruppe -$COR_{21}$ oder -$CON(R_{19})(R_{20}$ und $R_{24}$ $C_1$-$C_{12}$ Alkyl, Phenyl oder $C_7$-$C_{10}$ Alkylphenyl bedeuten,

Y -O- oder -$N(R_{25})$-, worin $R_{25}$ Wasserstoff, $C_1$-$C_{18}$ Alkyl, $C_3$-$C_{12}$-Alkenyl, $C_3$-$C_{12}$ Cycloalkyl, Phenyl, $C_7$-$C_{14}$ Aralkyl, $C_7$-$C_{14}$ Alkaryl, $C_2$-$C_{11}$ Alkoxyalkyl oder eine Gruppe der Formel IV ist, bedeutet,

Z -O- oder-$N(R_{26})$- ist, worin $R_{26}$ Wasserstoff, $C_1$-$C_{18}$ Alkyl, $C_3$-$C_{12}$-Alkenyl, $C_3$-$C_{12}$ Cycloalkyl, Phenyl, $C_7$-$C_{14}$ Alkaryl, $C_7$-$C_{14}$ Aralkyl, $C_2$-$C_{11}$ Alkoxyalkyl, eine Gruppe -$COR_{27}$, -$COOR_{28}$, -$CON(R_{29})(R_{30})$, -$CH_2$-$CH(R_{31})$-$OR_{32}$, -$SOR_{33}$ oder -$SO_2R_{33}$ ist, worin $R_{27}$ eine der für $R_{21}$ angegebenen Bedeutungen hat oder ein heterozyklischer Ring ist, $R_{28}$ eine der für $R_{18}$ angegebenen Bedeutungen hat, $R_{29}$ eine der für $R_{19}$ angegebenen Bedeutungen hat, $R_{30}$ eine der für $R_{20}$ angegebenen Bedeutungen hat, $R_{31}$ eine der für $R_{22}$ angegebenen Bedeutungen hat und $R_{32}$ eine der für $R_{23}$ angegebenen Bedeutungen hat und $R_{33}$ eine der für $R_{24}$ angegebenen Bedeutungen hat, oder $R_{26}$ eine der Gruppen

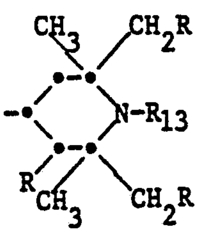 , 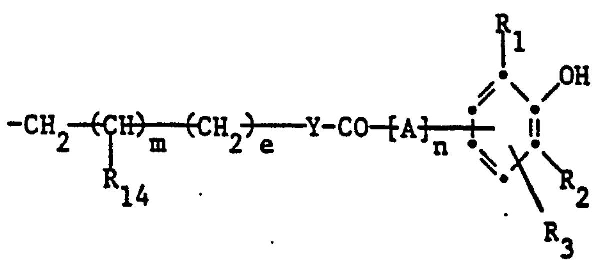

oder

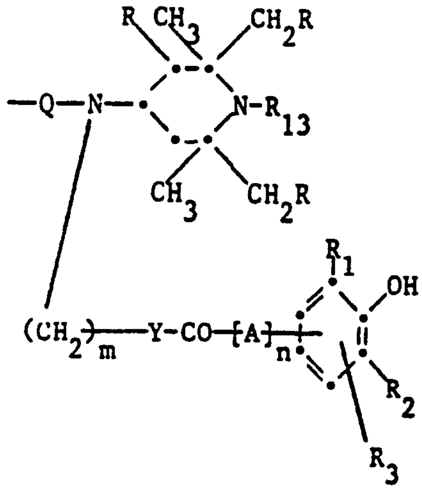

bedeutet,

Q eine Gruppe -(-$C_bH_{2b}$-)-, worin b eine Zahl 2 bis 12 bedeutet, $C_4$-$C_8$ Alkenylen, $C_5$-$C_{12}$ Cycloalkylen, Phenylen, Xylylen, Bitolylen, eine Gruppe -CO-(-$C_rH_{2r}$-)-CO-,

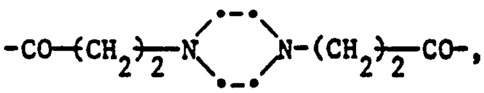, 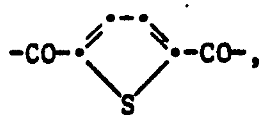, 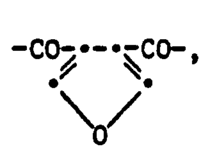,

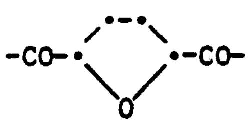, 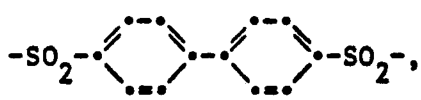,

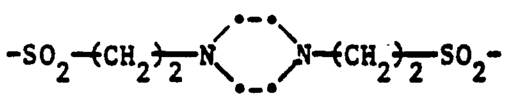 oder eine Gruppe

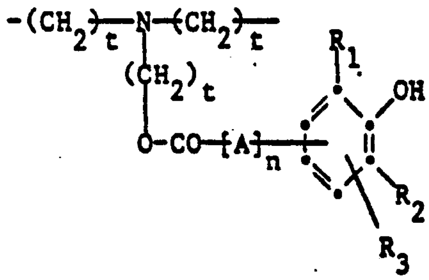

worin t eine Zahl 0 bis 5 ist, bedeutet,

$R_{13}$ eine Gruppe der Formel

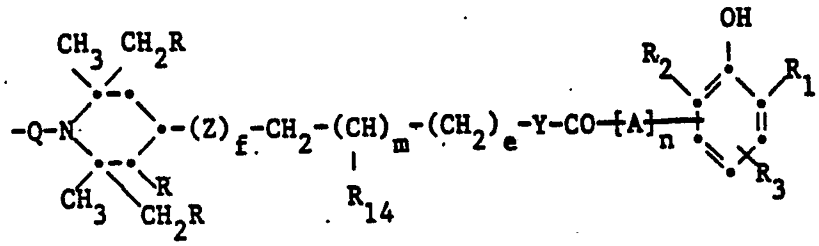

ist, und

$R_{14}$ Wasserstoff, $C_1$-$C_{18}$ Alkyl, $C_7$-$C_{23}$ Phenoxyalkyl, Phenyl, $C_7$-$C_{14}$-Aralkyl, $C_2$-$C_{11}$ Alkoxyalkyl oder eine Gruppe

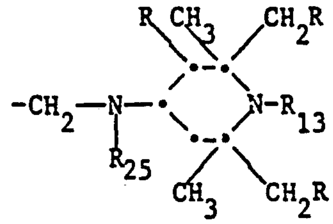

ist,

$R_{15}$ Wasserstoff, Methyl, Ethyl, Phenyl, Phenoxymethyl,

$R_{16}$ Wasserstoff, $-OR_{34}$, $-OCOR_{35}$, $-N(R_{36})-COR_{35}$, $-OSO_2R_{35}$, $-N(R_{36})-SO_2R_{35}$ ist, wobei $R_{34}$ Wasserstoff, $C_1$-$C_{12}$ Alkyl, Allyl, Benzyl ist, $R_{35}$ Wasserstoff, $C_1$-$C_{12}$ Alkyl, $C_2$-$C_6$ Alkenyl, Chlormethyl, $C_5$-$C_8$ Cycloalkyl, $C_7$-$C_9$ Phenylalkyl, $C_7$-$C_{10}$ Alkylphenyl, Phenyl oder eine Gruppe

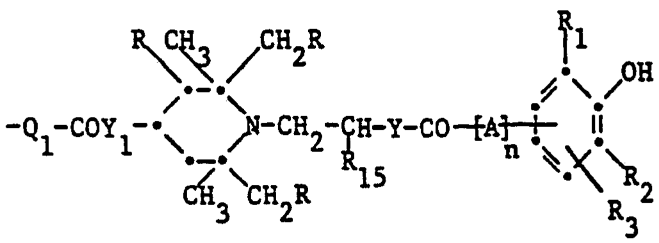

bedeutet, worin

$Y_1$ eine der für Y angegebenen Bedeutungen hat,

$Q_1$ eine Gruppe $-(-C_rH_{2r}-)-$, $C_4$-$C_8$ Alkenylen, $C_5$-$C_{12}$ Cycloalkylen, Phenylen, Xylylen, Bitolylen, eine Gruppe

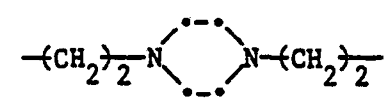 , 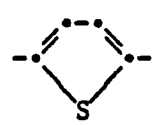 , 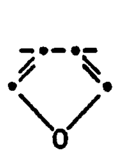 oder 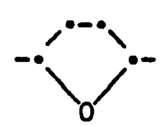 ist,

und $R_{36}$ Wasserstoff, $C_1$-$C_{12}$ Alkyl, $C_5$-$C_8$ Cycloalkyl oder Benzyl bedeutet und

$R_{17}$ Wasserstoff, Cyano, $-COOR_{18}$, $-CONH_2$, $-CON(R_{19})(R_{20})$ oder $-CH_2NHR_{37}$, worin $R_{37}$ eine Gruppe $-COR_{21}$, $-COOR_{18}$, $-CON(R_{19})(R_{20})$, $-CH_2-CH(R_{22})-OR_{23}$, $-SOR_{24}$, $-SO_2R_{24}$ ist, bedeutet, wobei die wiederholt erwähnten Reste und Symbole immer die erstmals angegebene Bedeutung haben.

Bedeuten etwaige Substituenten Alkyl, so handelt es sich um geradkettige oder verzweigte Alkylgruppen. Bedeuten sie $C_1$-$C_4$ Alkyl, dann handelt es sich um Methyl, Ethyl, n-Propyl, Isopropyl, n-Butyl, sec.-Butyl oder tert.-Butyl. Bedeuten sie $C_1$-$C_8$ Alkyl, so kommen zusätzlich z. B. n-Pentyl, 2,2-Dimethylpropyl, n-Hexyl, 2,3-Dimethylbutyl, n-Octyl oder 1,1,3,3-Tetramethylbutyl in Frage. Bedeuten sie $C_1$-$C_{12}$ Alkyl, so können sie zusätzlich auch z. B. Nonyl, Decyl, Undecyl und Dodecyl sein. Als $C_1$-$C_{18}$ Alkyl bedeuten sie zusätzlich z. B. Tetradecyl, Hexadecyl, Heptadecyl oder Octadecyl.

Bedeuten etwaige Substituenten $C_5$-$C_8$ Cycloalkyl, so handelt es sich z. B. um Cyclopentyl, Cyclohexyl, Cycloheptyl, α-Methylcyclohexyl, Cyclooctyl oder Dimethylcyclohexyl. Als $C_3$-$C_{12}$ Cycloalkyl können sie zusätzlich z. B. Cyclopropyl, Cyclononyl, Cyclodecyl oder Cyclododecyl sein. Bevorzugt ist Cyclohexyl.

$R_{11}$ und $R_{35}$ bedeuten als $C_7$-$C_9$ Phenylalkyl beispielsweise Benzyl, Phenylethyl oder Phenylpropyl. Bedeuten

etwaige Substituenten $C_7$-$C_{14}$ Aralkyl, so handelt es sich darüber hinaus auch z. B. um Phenylbutyl oder Naphthylmethyl.

Bedeuten etwaige Substituenten $C_7$-$C_{10}$ Alkylphenyl, so können sie beispielsweise Tolyl, Xylyl, Isopropylphenyl, tert.-Butylphenyl oder Diethylphenyl sein.

$R_{13}$ ist als $C_3$-$C_6$ Alkenylmethyl, z. B. Allyl, Methallyl, Dimethylallyl oder 2-Hexenyl. $R_{21}$, $R_{27}$ und $R_{35}$ können als $C_2$-$C_6$ Alkenyl zusätzlich auch Vinyl sein.

G, T, $R_{25}$ und $R_{26}$ können als $C_3$-$C_{12}$ Alkenyl beispielsweise Allyl, Methallyl, 2-Butenyl, 2-Hexenyl, 2-Octenyl, 4-Octenyl, 2-Decenyl oder 2-Dodecenyl sein. Bevorzugt ist Allyl.

G und T als $C_3$-$C_4$ Alkinyl, sowie $R_{13}$ als $C_3$-$C_4$ Alkinylmethyl, bedeuten z. B. Propargyl, n-But-4-inyl oder n-But-2-inyl. Bevorzugt ist Propargyl.

Bedeuten etwaige Substituenten $C_7$-$C_{14}$ Alkaryl so handelt es sich z. B. um durch $C_1$-$C_4$ Alkyl substituiertes Phenyl, wie p-Tolyl, 2,4-Dimethylphenyl, 2,6-Dimethylphenyl, 2,4-Diethylphenyl, 2,6-Diethylphenyl, 4-tert.-Butylphenyl, 2,4-Di-tert-butylphenyl oder 2,6-Di-tert.-butylphenyl. Bevorzugt sind 2,4-Di-tert.-butylphenyl und 2,4-Dimethylphenyl.

$R_{22}$ und $R_{31}$ bedeuten als $C_3$-$C_4$ Alkoxyalkyl z. B. Ethoxymethyl, 2-Methoxyethyl oder 2-Ethoxyethyl. $R_{14}$, $R_{25}$ und $R_{26}$ können als $C_2$-$C_{11}$ Alkoxyalkyl darüber hinaus auch Methoxymethyl, 2-n-Butoxyethyl, 2-n-Butoxypropyl, 2-n-Octoxyethyl, 3-n-Octoxypropyl oder 6-n-Butoxyhexyl sein.

$R_{14}$ ist als $C_7$-$C_{23}$ Phenoxyalkyl z. B. Phenoxymethyl, Phenoxyethyl, Phenoxypropyl, Phenoxybutyl, Phenoxyoctyl, Phenoxydecyl, Phenoxydodecyl oder Phenoxyhexadecyl.

Sind Q und $Q_1$ $C_4$-$C_8$ Alkenylen, so handelt es sich z. B. um 2-Butenylen-1,4.

Bedeuten Q und $Q_1$ $C_5$-$C_{12}$ Cycloalkylen, so handelt es sich z. B. um Cyclopentylen, Cyclohexylen, Cyclooctylen, Cyclodecylen oder Cyclododecylen. Bevorzugt ist Cyclohexylen.

Bedeuten etwaige Substituenten Halogen, so handelt es sich z. B. um Brom, Iod und insbesondere Chlor.

Als durch Halogen, Cyano, $-COOR_{18}$ oder $-CON(R_{19})(R_{20})$ substituiertes $C_1$-$C_4$ Alkyl bedeutet $R_{13}$ beispielsweise Chlormethyl, 1-Chlorethyl, 2-Chlorethyl, 3-Chlorpropyl, 4-Chlorbutyl, Brommethyl, Iodmethyl, Cyanomethyl, Methoxycarbonylmethyl, N,N'-Dimethylaminocarbonylmethyl oder 2-Chlor-2-methylpropyl.

In der Bedeutung als durch Phenoxy, Benzyloxy, Cyclohexyloxy oder Cyano substituiertes $C_1$-$C_8$ Alkyl kann G beispielsweise einer der folgenden Reste sein: 2-Phenoxyethyl, 2-Benzyloxyethyl, Cyclohexyloxymethyl, 2-Cyanoethyl, Cyanomethyl oder 3-Cyanopropyl.

G und T bedeuten als $C_6$-$C_{18}$ Alkylcycloalkyl z. B. Methylcyclohexyl, Ethylcyclohexyl, Butylcyclohexyl, tert.-Butylcyclohexyl, Dodecylcyclohexyl, Ethylcyclopentyl oder Butylcyclopentyl und als $C_6$-$C_{14}$ Cycloalkylalkyl beispielsweise Cyclohexylmethyl, Cyclohexylethyl, Cyclohexylbutyl, Cyclohexylhexyl, Cyclohexyloctyl, Cyclopentylpropyl oder Cyclopentylhexyl.

Als durch $C_7$-$C_{10}$ Alkylphenoxy substituiertes $C_1$-$C_{18}$ Alkyl kann G beispielsweise p-Methylphenoxymethyl, p-Methylphenoxyethyl, P-Methylphenoxypropyl, p-tert.-Butylphenoxymethyl, p-tert.-Butylphenoxyethyl, 2,4-Dimethylphenoxymethyl, 2,4-Dimethylphenoxyethyl, 2,4-Di-tert.-butylphenoxyethyl, 2,6-Di-tert.-butylphenoxymethyl oder 2,4,6-Tri-methylphenoxyethyl sein.

In der Bedeutung als durch -O-, -S-, -SO- oder $-SO_2-$ unterbrochenes $C_2$-$C_{18}$ Alkyl sind G und T z. B. einer der folgenden Reste: Methoxymethyl, 2-Butoxyethyl, 2-Octyloxyethyl, Isopropyloxymethyl, 3-Butylthio-Propyl, 2-Decylthioethyl, 2-(Isohexylsulfinyl)-ethyl, 2-(Butyl-sulfonyl)-ethyl oder 3-(Ethylsulfonyl)-propyl.

$R_{21}$ und $R_{27}$ bedeuten als durch 1 oder 2 $C_1$-$C_4$ Alkyl und 1 Hydroxy substituiertes Phenyl, Phenylmethyl oder Phenylethyl z. B. 2,5-Dimethyl-4-hydroxyphenyl, 3,5-Di-tert.butyl-4-hydroxyphenyl, 3,5-Dimethyl-4-hydroxybenzyl, 3,5-Di-tert.butyl-4-hydroxybenzyl oder 2-(3,5-Di-tert.-butyl-4-hydroxyphenyl)-ethyl.

Bedeuten D, Q oder $Q_1$ eine Gruppe $-C_2H_{2r}-$, worin r eine Zahl zwischen 1 und 12 ist, so bedeutet r bevorzugt eine Zahl zwischen 2 und 8. Beispiele sind Methylen, Ethylen, Trimethylen, Tetramethylen, Hexamethylen, Octamethylen, Nonamethylen, 2,2,4-Trimethylhexamethylen, Decamethylen, Dodecamethylen.

$R_{27}$ bedeutet als heterozyklischer Ring beispielsweise Pyridin, Chinolin, Pyrimidin, Thiazol, Imidazol, Oxazol, Pyrrolidin, Piperazin, Morpholin, Piperidin, Furan, Tetrahydrofuran, Thiophen, Pyrrol oder Indol.

Bevorzugt werden solche farbphotographischen Aufzeichnungsmaterialien, die als Stabilisator mindestens eine Verbindung der Formel I enthalten, worin A eine Gruppe $-CH_2-$, $-CH_2-CH(R_5)-$,

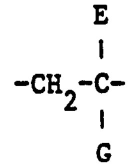 oder 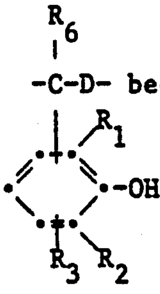 bedeutet.

Bevorzugt werden solche farbphotographische Aufzeichnungsmaterialien, die als Stabilisator mindestens eine Verbindung der Formel V

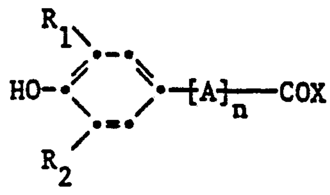

(V)

enthalten, worin

n die Zahlen 0 oder 1 bedeutet,

$R_1$ Wasserstoff oder $C_1$-$C_4$ Alkyl ist,

$R_2$ $C_1$-$C_4$ Alkyl bedeutet,

A eine Gruppe $-CH_2-$, $-CH_2-CH(R_5)-$,

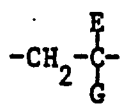 oder 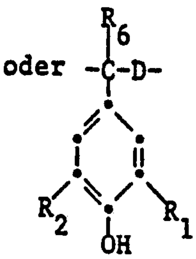

ist, worin $R_5$ und $R_6$ Wasserstoff oder Methyl sind, E Cyano, $-COCH_3$ und $-COOCH_3$, G Wasserstoff $C_1$-$C_{18}$ Alkyl, Allyl, Cyclohexyl, Benzyl oder eine Gruppe

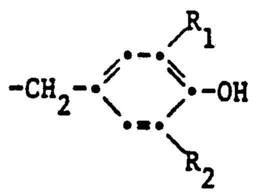 ist oder E und G zusammen

eine Gruppe $-CO-(CH_2-)_4$ bilden, D eine Gruppe $-(-C_rH_{2r}-)-$ ist, worin r die Zahlen 0 bis 6 bedeutet, und

X eine Gruppe der Formel

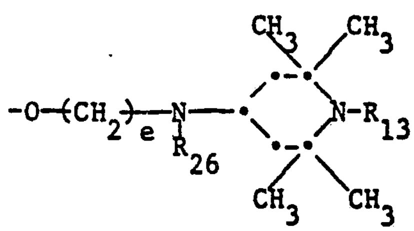

oder der Formel

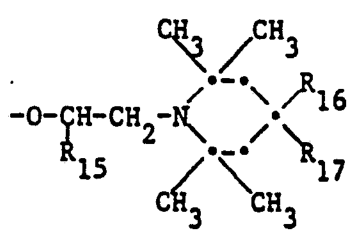

ist, worin

e die Zahlen 2 bis 3 bedeutet,

$R_{13}$ Hydroxy, Methyl, Allyl, Benzyl, 2-Hydroxyethyl, Acetyl, Acroyl oder eine Gruppe der Formel $-CON(R_{19})(R_{20})$, worin $R_{19}$ $C_1$-$C_8$ Alkyl, Cyclohexyl oder Phenyl und $R_{20}$ Wasserstoff oder $C_1$-$C_{12}$ Alkyl sind, bedeutet,

$R_{26}$ Wasserstoff, $C_1$-$C_8$ Alkyl, eine Gruppe $-COR_{27}$, $-COOR_{28}$, $-CON(R_{29})(R_{30})$, $-CH_2-CH(R_{31})-OR_{32}$ oder $-SO_2R_{33}$ ist, worin $R_{27}$ $C_1$-$C_{12}$ Alkyl, $C_2$-$C_4$ Alkenyl, Cyclohexyl, Benzyl, Phenyl, 2-(3,5-Di-tert.-butyl-4-hydroxyphenyl)-ethyl oder ein heterozyklischer Ring,

$R_{28}$ $C_1$-$C_8$ Alkyl, Allyl oder Cyclohexyl,
$R_{29}$ $C_1$-$C_{12}$ Alkyl, Cyclohexyl oder Phenyl,
$R_{30}$ Wasserstoff oder $C_1$-$C_{12}$ Alkyl sind oder
$R_{29}$ und $R_{30}$ zusammen mit dem N-Atom, an das sie gebunden sind, einen 6-gliedrigen heterocyclischen Ring bilden,
$R_{31}$ Wasserstoff, Methyl oder Phenyl und
$R_{32}$ Wasserstoff, $C_1$-$C_4$ Alkyl, eine Gruppe $-COR_{21}$ oder $-CON(R_{19})(R_{20})$ und
$R_{33}$ $C_1$-$C_{12}$ Alkyl, Phenyl oder $C_7$-$C_{10}$ Alkylphenyl sind,
$R_{15}$ Wasserstoff, Methyl oder Phenyl ist,
$R_{16}$ Wasserstoff, $-OR_{34}$, $-OCOR_{35}$ oder $-N(R_{36})-COR_{35}$ ist, wobei $R_{34}$ Wasserstoff, $C_1$-$C_8$ Alkyl, Allyl, Benzyl oder Phenyl, $R_{35}$ Wasserstoff $C_1$-$C_8$ Alkyl, Allyl, Benzyl oder Phenyl bedeutet und $R_{36}$ Wasserstoff, $C_1$-$C_4$ Alkyl, Cyclohexyl oder Benzyl ist und
$R_{17}$ Wasserstoff, Cyano, $-COOR_{18}$, $-CONH_2$, $-CON(R_{19})(R_{20})$ oder $-CH_2NHR_{37}$, worin $R_{37}$ eine Gruppe $-COR_{21}$, $-COOR_{18}$, $-CON(R_{19})(R_{20})$, $-CH_2-CH(R_{22})-OR_{23}$ ist, bedeutet,

wobei die in dieser Bevorzugung wiederholt erwähnten Reste und Symbole immer die in dieser Bevorzugung erstmals angegebene Bedeutung haben.

Besonders bevorzugt werden farbphotographische Aufzeichnungsmaterialien, die als Stabilisator mindestens eine Verbindung der Formel V enthalten, worin
n die Zahlen 0 oder 1 bedeutet,
$R_1$ Wasserstoff, Methyl oder tert.-Butyl ist,
$R_2$ Methyl oder tert.-Butyl bedeutet,
A eine Gruppe $-CH_2-$ oder $-CH_2CH_2-$ ist, worin
X

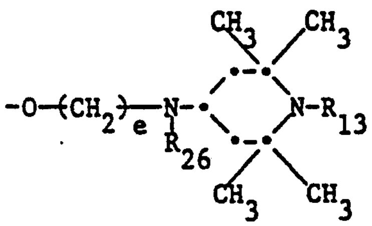 oder der Formel 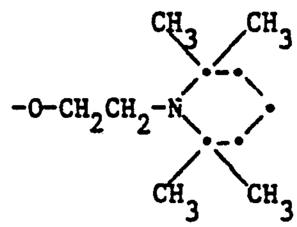

ist, worin e die Zahlen 2 bis 3 bedeutet,
$R_{13}$ Methyl, Allyl, Benzyl, 2-Hydroxyethyl, Acetyl oder Acroyl ist,
$R_{26}$ Wasserstoff, $C_1$-$C_8$ Alkyl oder eine Gruppe der Formel $-COR_{27}$ oder $-SO_2R_{33}$ ist, worin $R_{27}$ $C_1$-$C_{12}$ Alkyl oder $C_2$-$C_4$ Alkenyl ist, und $R_{33}$ $C_1$-$C_4$ Alkyl, Phenyl oder p-Tolyl ist.

Die Verbindungen der Formel I können in Analogie zu bekannten Verbindungen erhalten werden, wie sie z. B. in den DE-OS-2 456 364, 2 647 452, 2 654 058 und 2 656 769 beschrieben sind. Die Ausgangssubstanzen sind bekannt. Sollten einzelne von Ihnen noch neu sein, so können sie in Analogie zu den bekannten erhalten werden.

Die letzte Stufe der Synthese ist entweder eine direkte Veresterung (Säure + Alkohol oder Säurechlorid + Alkohol), eine Umesterung oder eine Amidierung.

Typische Vertreter von Verbindungen der Formel I sind in der nachstehenden Tabelle I aufgeführt.

**Tabelle I**

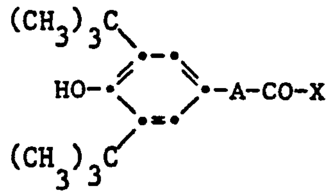

| Stabilisator Nr. | A | X |
|---|---|---|
| 1 | $-CH_2CH_2-$ | 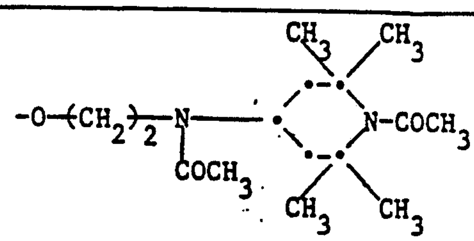 |
| 2 | 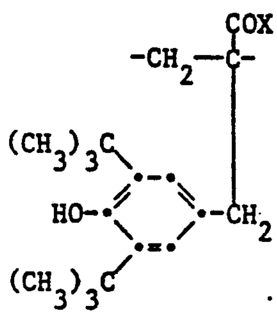 | 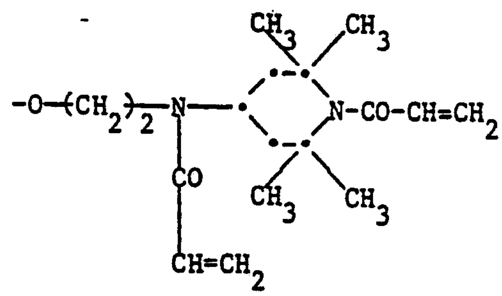 |
| 3 | 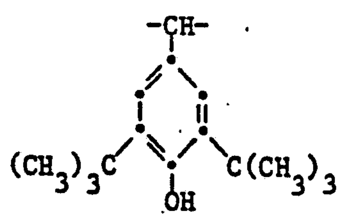 | 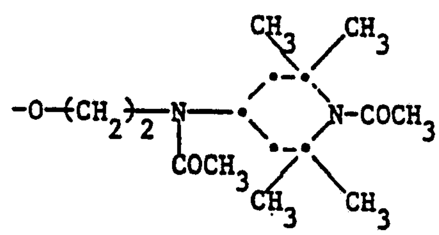 |
| 4 | 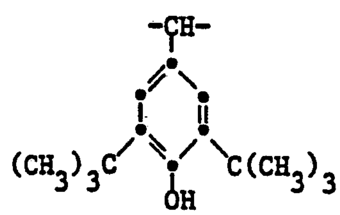 | 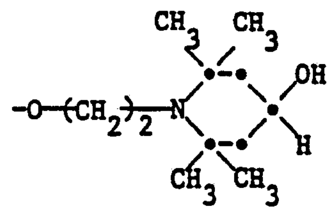 |
| 5 | $-CH_2-CH_2-$ | 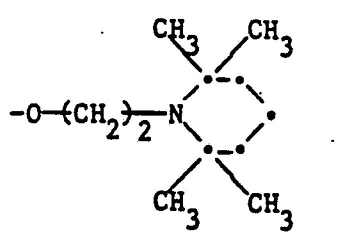 |

**Tabelle I** (Fortsetzung)

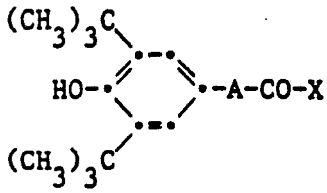

| Stabi-lisator Nr. | A | X |
|---|---|---|
| 6 | 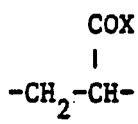 | 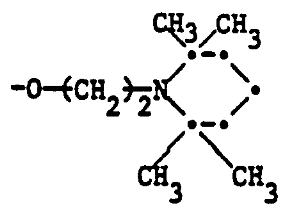 |
| 7 | $-CH_2CH_2-$ | 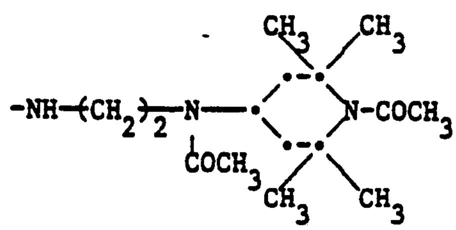 |
| 8 | $-CH_2CH_2-$ | 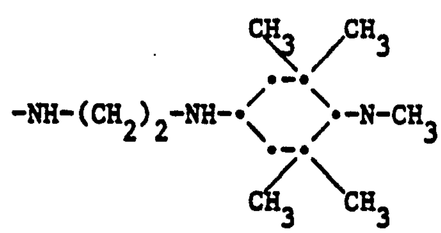 |
| 9 | --- | 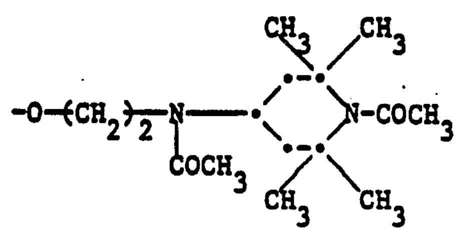 |
| 10 | --- | 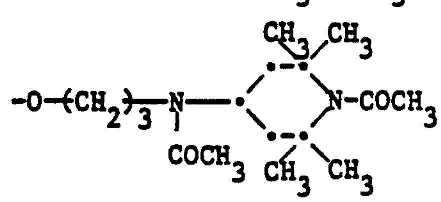 |
| 11 | --- | 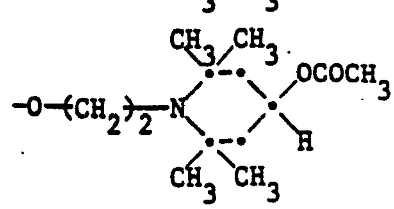 |

**Tabelle I** (Fortsetzung)

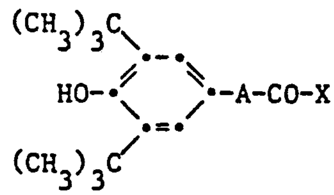

| Stabilisator Nr. | A | X |
|---|---|---|
| 12 | --- | 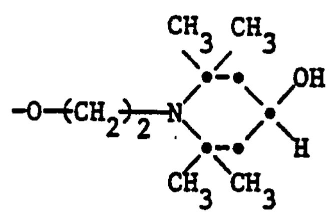 |
| 13 | $-CH_2CH_2-$ | 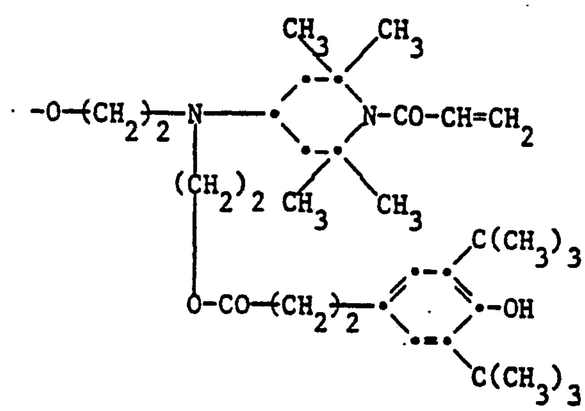 |
| 14 | $-CH_2CH_2-$ | 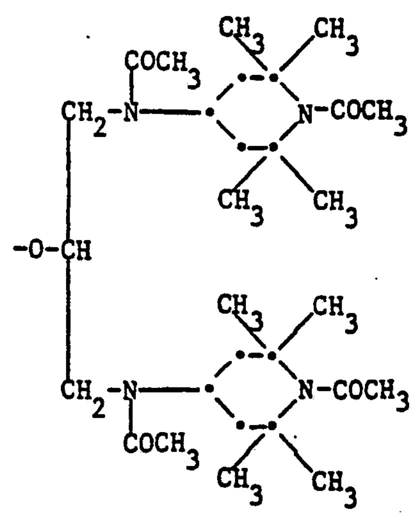 |

Weitere typische Vertreter von Verbindungen der Formel I sind die folgenden:

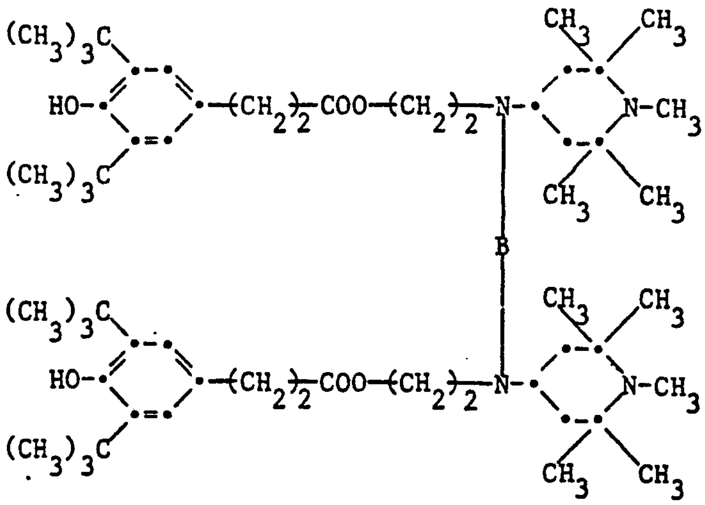

17) B = $-(CH_2)_6-$

18) B = $-CO-(CH_2)_4-CO-$

19) B = 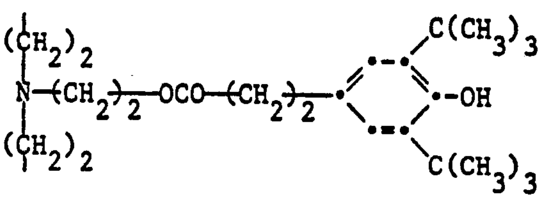

20) 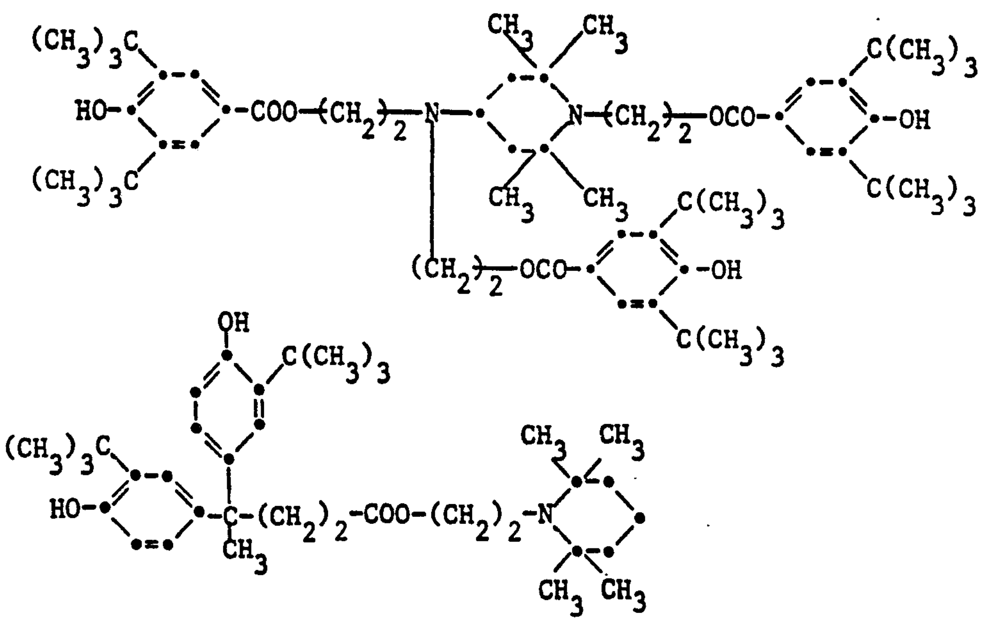

21)

22)

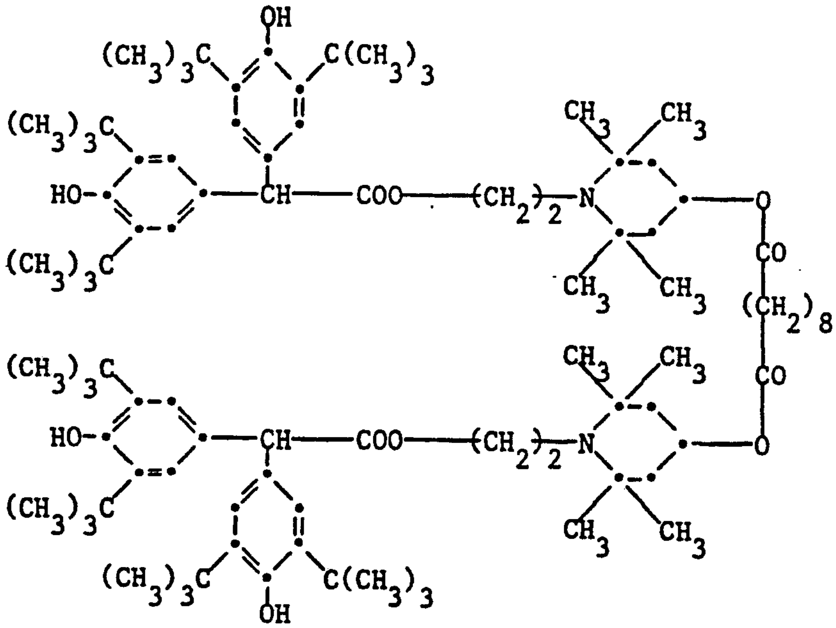

23)

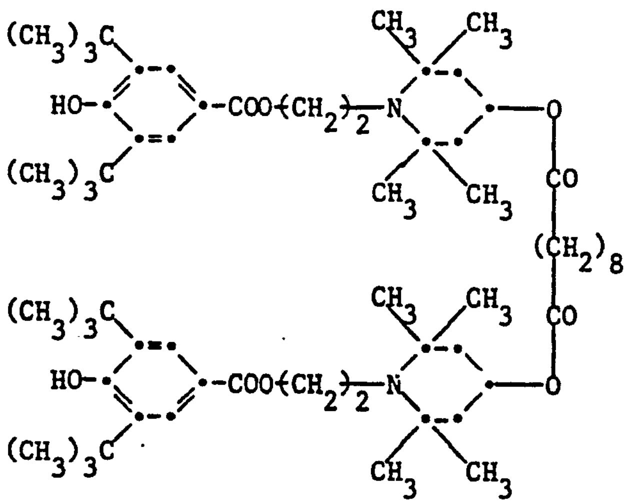

24)

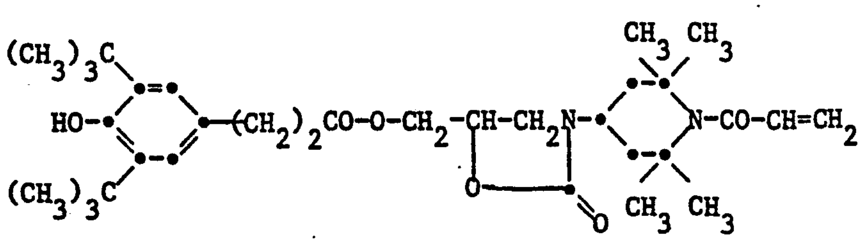

25)

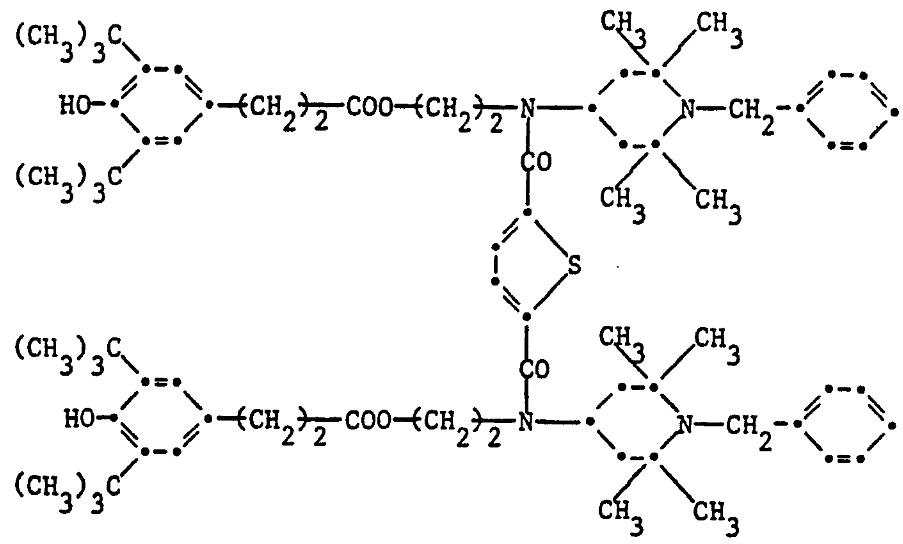

26)

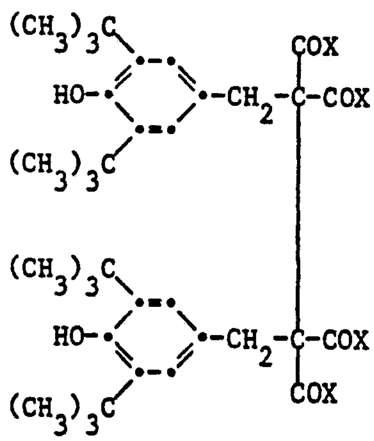

X =

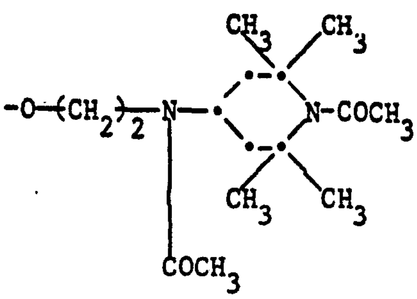

Die Stabilisatoren der Formel I können allein oder zusammen mit anderen Verbindungen in bekannter Weise in ein photographisches Material eingearbeitet werden.

In der Regel werden die Stabilisatoren allein oder zusammen mit anderen Verbindungen, insbesondere mit Farbkupplern, in Form einer Dispersion in das photographische Material eingearbeitet, wobei diese Dispersion entweder kein Lösungsmittel oder hoch- oder tiefsiedende Lösungsmittel oder ein Gemisch solcher Lösungsmittel enthält. Eine weitere geeignete Einarbeitungsform besteht darin, dass man die Stabilisatoren allein oder zusammen mit weiteren Verbindungen zusammen mit einem Polymer in Form eines Latex in das photographische Material einarbeitet.

Die Dispersionen werden dann zur Herstellung der Schichten von farbphotographischen Aufzeichnungsmaterialien verwendet. Diese Schichten können z. B. Zwischen- oder Schutzschichten, insbesondere jedoch lichtempfindliche (blau-, grün- und rot-empfindliche) Silberhalogenidemulsionsschichten sein, in denen bei der Entwicklung des belichteten Aufzeichnungsmaterials aus den entsprechenden Farbkupplern, die Blaugrün (Cyan)-, Purpur (Magenta)- und Gelbfarbstoffe gebildet werden.

Die Silberhalogenidschichten können beliebige Farbkuppler, insbesondere Blaugrün-, Purpur- und Gelb-Kuppler, die zur Bildung der genannten Farbstoffe und damit der Farbbilder verwendet werden, enthalten.

Da das Substrat die Wirkung und Stabilität der Verbindungen der Formel I beeinflusst, werden Substrate (Lösungsmittel, Polymere) bevorzugt, die zusammen mit diesen Verbindungen eine möglichst gute Beständigkeit der zu stabilisierenden Materialien ergeben.

In der Regel werden die Stabilisatoren der Formel I in Schichten eingearbeitet, die zusätzlich eine nach üblichen Methoden hergestellte und sensibilisierte Silberhalogenid-Dispersion enthalten. Sie können jedoch auch in zu Silberhalogenid enthaltenden Schichten benachbarten Schichten vorhanden sein.

Die erfindungsgemässen photographischen Materialien besitzen einen üblichen Aufbau und übliche Komponenten. Bevorzugt werden jedoch ein Aufbau und Komponenten, die die Wirksamkeit der Stabilisatoren der Formel I verstärken oder zumindest nicht nachteilig beeinflussen.

Im photographischen Aufzeichnungsmaterial gemäss vorliegender Erfindung können die Stabilisatoren gemäss Formel I, ausser mit den Farbkupplern zusätzlich auch mit Ultraviolettabsorbern, anderen Lichtschutzmitteln oder Stabilisatoren in der gleichen Schicht kombiniert werden.

Wenn die Diffusionstransfermethode angewendet wird, kann der Stabilisator auch in eine Empfangsschicht eingearbeitet werden.

Die erfindungsgemässen farbphotographischen Materialien können in bekannter Weise verarbeitet werden. Ferner können sie im Verlauf oder nach der Verarbeitung in einer Weise behandelt werden, die ihre Stabilität weiter erhöht, beispielsweise durch die Behandlung in einem Stabilisatorbad oder das Aufbringen eines Schutzüberzuges.

Die erfindungsgemäss einzusetzenden Stabilisatoren eignen sich in gewissen Fällen auch zum Schutz farbphotographischer Schichten, in denen die Farbstoffe direkt in die Emulsion eingelagert werden und das Bild durch selektive Bleichung erzeugt wird.

Die Menge des Stabilisators oder der Stabilisatoren kann in weiten Grenzen schwanken und liegt etwa im Bereich von 1 bis 2000 mg, vorzugsweise 100 bis 800 und insbesondere 200-500 mg pro $m^2$ der Schicht, in die es (sie) eingearbeitet wird (werden).

Falls das photographische Material ein UV-Strahlung absorbierendes Mittel enthält, so kann dieses mit dem Stabilisator zusammen in einer Schicht oder auch in einer benachbarten Schicht vorhanden sein. Die Menge des UV-Absorbers oder der UV-Absorber kann in weiten Grenzen schwanken und liegt etwa im Bereich von 200 - 2000 mg, vorzugsweise 400 - 1000 mg/$m^2$ der Schicht, in der er (sie) eingearbeitet wird (werden).

Beispiele für Ultraviolett-Absorber sind Verbindungen vom Benzophenon-, Acrylnitril-, Thiazolidon-, Benztriazol-, Oxazol-, Thiazol- und Imidazoltyp.

Die mit dem erfindungsgemässen Aufzeichungsmaterial durch Belichtung und Entwicklung erhaltenen Farbbilder zeigen eine sehr gute Lichtechtheit gegenüber sichtbarem und ultraviolettem Licht. Die Verbindungen der Formel I sind praktisch farblos, so dass es zu keiner Verfärbung der Bilder kommt; ausserdem sind sie gut verträglich mit den üblichen, in den einzelnen Schichten vorhandenen photographischen Zusatzstoffen. Aufgrund ihrer guten Wirksamkeit kann man ihre Einsatzmenge herabsetzen und vermeidet so ihre Ausfällung oder ihr Auskristallisieren, wenn man sie als organische Lösung in die wässerigen Bindemittelemulsionen, die für die Herstellung photographischer Schichten verwendet werden, einarbeitet. Die einzelnen nach der Belichtung des photographischen Aufzeichnungsmaterials notwendigen Verarbeitungsschritte zur Herstellung der Farbbilder werden durch die Stabilisatoren der Formel I nicht nachteilig beeinflusst. Ferner kann die bei blauempfindlichen Emulsionen häufig auftretende sogenannte Druckschleierbildung weitgehend zurückgedrängt werden. Diese kann z. B. auftreten, wenn auf photographische Materialien (Silberhalogenidemulsionsschichten, die auf einem Träger aus natürlichen oder synthetischen Materialien befinden) mechanische Beanspruchungen, z. B. Drehen, Biegen oder Reiben, während der Herstellung oder während der Behandlung vor der Entwicklung ausgeübt werden. (T.H. James, The Theory of Photographic Process 4. Auflage, Macmillan, New York, N.Y. 1977, Seite 23 ff., S. 166 ff.).

**Anwendungsbeispiele**

0,087 g des Gelbkupplers der Formel

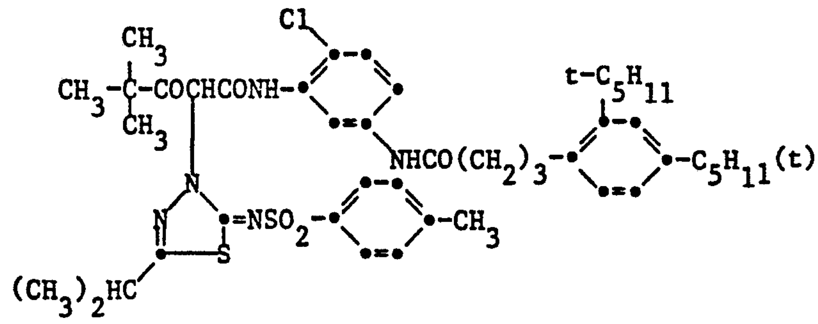

und 0,026 g eines der in den nachfolgenden Tabellen angegebenen Lichtschutzmittels werden in 2,0 ml eines Gemisches von Trikresylphosphat/Ethylacetat (1,5 g in 100 ml) gelöst. Zu dieser Lösung gibt man 7,0 ml einer 6 %-igen Gelatinelösung, 0,5 ml einer 8 %-igen Lösung des Netzmittels der Formel

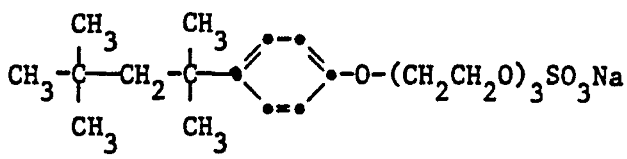

in Isopropanol/Wasser (3:4) und 0,5 ml Wasser und emulgiert mit Ultraschall bei einer Leistung von 100 Watt während 5 Minuten.

Zu 2,5 ml der so erhaltenen Emulsion gibt man 2,0 ml einer Silberbromid-Emulsion mit einem Silbergehalt von 6,0 g pro Liter, 0,7 ml einer 1 %-igen wässerigen Lösung des Härters der Formel

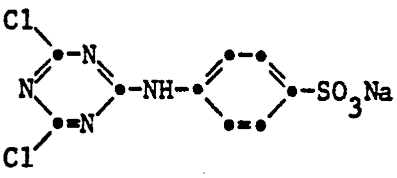

und 3,8 ml Wasser, stellt das Gemisch auf einen pH-Wert von 6,5 und vergiesst es auf ein auf eine Glasplatte aufgezogenes substriertes kunststoffbeschichtetes, weisses Papier.

Nach dem Erstarren wird in einem Trockenschrank mit Umluft bei Raumtemperatur getrocknet.

Nach 7 Tagen werden auf 35 x 180 mm geschnittene Proben hinter einem Stufenkeil mit 3000 Lux·s belichtet und anschliessend im Ektaprint 2®-Prozess der Firma Kodak verarbeitet.

Die so erhaltenen Gelbkeile werden in einem Atlas Weather-Ometer mit einer 2500 W-Xenonlampe mit total 42 kJoule/$cm^2$ bestrahlt (eine Vergleichsprobe enthält kein Lichtschutzmittel).

Der dabei eingetretene Farbdichteverlust wird bestimmt durch Messung der Farbdichte bei λ max. mit einem Densitometer® TR 924 A der Firma Macbeth.

Die Ergebnisse sind in der folgenden Tabelle eingeführt:

| Lichtschutzmittel Nr. | Dichteverlust an Maximum in Remission in Prozent |
|---|---|
| - | 36 |
| 1 | 17 |
| 9 | 17 |
| 15 | 15 |
| 16 | 15 |
| 20 | 18 |

**Patentansprüche**

1. Farbphotographisches Aufzeichnungsmaterial, das in mindestens einer lichtempfindlichen Silberhalogenidemulsionsschicht, einer Zwischenschicht und/oder einer Schutzschicht mindestens eine

Polyalkylpiperidinverbindung als Stabilisator enthält, dadurch gekennzeichnet, dass die Polyalkylpiperidinverbindung der Formel I

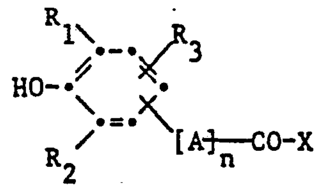

(I)

entspricht, worin

n die Zahlen 0 oder 1 bedeutet,

$R_1$ Wasserstoff, $C_1$-$C_8$ Alkyl, $C_5$-$C_8$ Cycloaklyl, Phenyl, $C_7$-$C_{14}$ Aralkyl, $C_7$-$C_{14}$ Alkaryl oder eine Gruppe

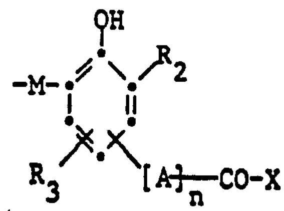

$R_2$ $C_1$-$C_8$ Alkyl, $C_5$-$C_8$ Cycloalkyl, Phenyl, $C_7$-$C_{14}$ Aralkyl, $C_7$-$C_{14}$ Alkaryl und

$R_3$ Wasserstoff oder Methyl sind,

M eine direkte Bindung, -S-, -S-S-, -S(O)-, $-S(O)_2-$ oder $-CH(R_4)-$, worin $R_4$ Wasserstoff oder $C_1$-$C_8$ Alkyl ist, bedeutet,

A eine Gruppe $-CH_2-$ $-CH_2-CH(R_5)-$,

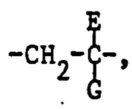 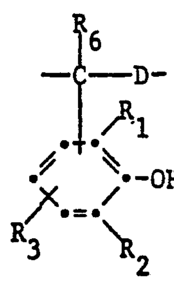 oder 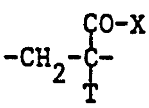

ist, worin

$R_5$ Wasserstoff, Methyl, Ethyl, Phenoxymethyl oder Phenyl ist,

$R_6$ Wasserstoff, $C_1$-$C_{12}$ Alkyl, $C_5$-$C_8$ Cycloalkyl, Phenyl oder Benzyl ist,

E -CN oder eine Gruppe $-COOR_7$, $-COR_8$, $-SO_2R_8$, $-P(O)(OR_9)_2$, -CHO, wobei $R_7$ $C_1$-$C_4$ Alkyl, $R_8$ $C_1$-$C_{12}$ Alkyl, $C_7$-$C_{14}$ Alkaryl, Phenyl und $R_9$ $C_1$-$C_{18}$ Alkyl, Phenyl oder Allyl sind,

G Wasserstoff, $C_1$-$C_{18}$ Alkyl, $C_3$-$C_{12}$ Alkenyl, $C_3$-$C_4$ Alkinyl, $C_3$-$C_{12}$ Cycloalkyl, $C_6$-$C_{18}$ Alkylcycloalkyl, $C_6$-$C_{14}$ Cycloalkylalkyl, $C_7$-$C_{14}$ Aralkyl, $C_7$-$C_{14}$ Alkaryl, Phenyl, durch Phenoxy, $C_7$-$C_{10}$ Alkylphenoxy, Benzyloxy, Cyclohexyloxy, Cyano, $-COOR_{10}$, $-OCOR_{11}$ oder $-P(O)(OR_{12})_2$ substituiertes $C_1$-$C_{18}$ Alkyl ist, wobei $R_{10}$ $C_1$-$C_{18}$ Alkyl, $C_3$-$C_{12}$ Cycloalkyl oder eine Gruppe der Formel II

$-(-C_aH_{2a}-)-CO-X$ (II),

worin a die Zahlen 1 bis 6 bedeutet,

$R_{11}$ $C_1$-$C_{18}$ Alkyl, $C_3$-$C_{12}$ Cycloalkyl, Phenyl, $C_7$-$C_9$ Phenylalkyl oder eine Gruppe der Formel III

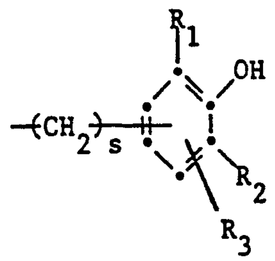

(III)

worin

s die Zahlen 0, 1 oder 2 bedeutet und

$R_{12}$ $C_1$-$C_8$ Alkyl, Allyl oder Phenyl sind, oder

G durch -O-, -S-, -SO-, -$SO_2$- unterbrochenes $C_2$-$C_{18}$ Alkyl oder eine Gruppe der Formel III ist, oder, wenn E eine Gruppe -$COR_8$ ist, G und $R_8$ zusammen unsubstituiertes oder durch Hydroxy oder Oxo substituiertes Tri- oder Tetramethylen bilden,

D eine Gruppe -(-$C_rH_{2r}$-)- oder

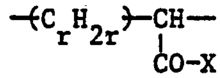

ist, worin

r die Zahlen 0 bis 12 bedeutet,

T Wasserstoff, $C_1$-$C_{18}$ Alkyl, $C_3$-$C_{12}$ Alkenyl, $C_3$-$C_4$ Alkinyl, $C_3$-$C_{12}$ Cycloalkyl, $C_6$-$C_{18}$ Alkylcycloalkyl, $C_6$-$C_{14}$ Cycloalkylalkyl, $C_7$-$C_{14}$ Aralkyl, $C_7$-$C_{14}$ Alkaryl, Phenyl, Cyano oder eine Gruppe -$COR_8$, -$SO_2R_8$ oder -$P(O)(OR_9)_2$, eine Gruppe der Formel III, eine Gruppe der Formel IV

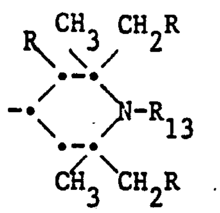

(IV)

oder eine Gruppe der Formel

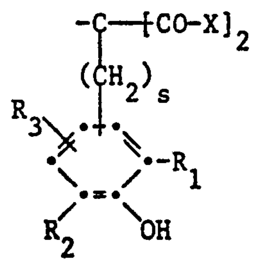

oder T durch -O-, -S-, -SO- oder -$SO_2$- unterbrochenes $C_1$-$C_{18}$ Alkyl ist,

X eine Gruppe der Formel

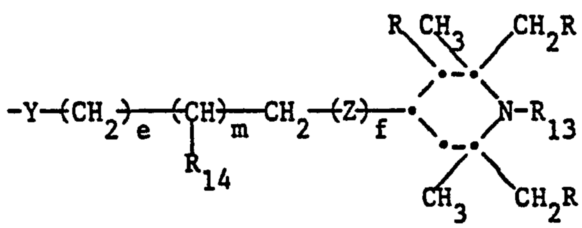

oder der Formel

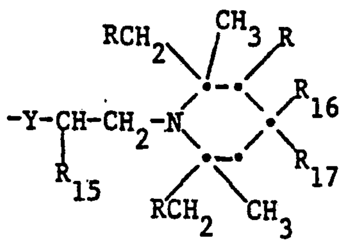

ist, worin
e die Zahlen 0 bis 5 und
f und m unabhängig voneinander die Zahlen 0 oder 1 bedeuten

R Wasserstoff oder Methyl ist
$R_{13}$ Hydroxy, $C_1$-$C_{12}$ Alkyl, $C_3$-$C_6$ Alkenylmethyl, $C_3$-$C_4$ Alkinylmethyl, $C_7$-$C_{14}$ Aralkyl, Glycidyl, durch Halogen, Cyano, $-COOR_{18}$ oder $-CON(R_{19})(R_{20})$ substituiertes $C_1$-$C_4$ Alkyl, eine Gruppe $-COR_{21}$, $-COOR_{18}$ oder $-CON(R_{19})(R_{20})$, eine Gruppe $-CH_2-CH(R_{22})-OR_{23}$, $-SO_{24}$, $-SO_2R_{24}$, $-OR_{18}$, $-OCOR_{21}$ ist, wobei $R_{18}$ $C_1$-$C_{12}$ Alkyl, Allyl, Cyclohexyl oder Benzyl, $R_{19}$ $C_1$-$C_{12}$ Alkyl, Allyl, Cyclohexyl, Benzyl, Phenyl oder $C_7$-$C_{10}$ Alkylphenyl, $R_{20}$ Wasserstoff, $C_1$-$C_{12}$ Alkyl, Allyl, Cyclohexyl oder Benzyl sind oder $R_{19}$ und $R_{20}$ zusammen mit dem N-Atom an das sie gebunden sind einen 5- oder 6-gliedrigen heterozyklischen Ring bilden, $R_{21}$ Wasserstoff, $C_1$-$C_{12}$ Alkyl, $C_2$-$C_6$ Alkenyl, Chloromethyl, $C_5$-$C_8$ Cycloalkyl, $C_7$-$C_{14}$ Aralkyl, Phenyl, $C_7$-$C_{10}$ Alkylphenyl oder durch 1 oder 2 $C_1$-$C_4$ Alkyl und 1 Hydroxy substituiertes Phenyl, Phenylmethyl oder Phenylethyl, $R_{22}$ Wasserstoff, $C_1$-$C_4$ Alkyl, $C_3$-$C_4$ Alkoxyalkyl, Phenyl oder Phenoxymethyl, $R_{23}$ Wasserstoff, $C_1$-$C_{12}$ Alkyl, eine Gruppe $-COR_{21}$ oder $-CON(R_{19})(R_{20})$ und $R_{24}$ $C_1$-$C_{12}$ Alkyl, Phenyl oder $C_7$-$C_{10}$ Alkylphenyl bedeuten,
Y -O- oder $-N(R_{25})-$, worin $R_{25}$ Wasserstoff, $C_1$-$C_{18}$ Alkyl, $C_3$-$C_{12}$ Alkenyl, $C_3$-$C_{12}$ Cycloalkyl, Phenyl, $C_7$-$C_{14}$ Aralkyl, $C_7$-$C_{14}$ Alkaryl, $C_2$-$C_{11}$ Alkoxyalkyl oder eine Gruppe der Formel IV ist, bedeutet,
Z -O- oder $-N(R_{26})-$ ist, worin $R_{26}$ Wasserstoff, $C_1$-$C_{18}$ Alkyl, $C_3$-$C_{12}$-Alkenyl, $C_3$-$C_{12}$ Cycloalkyl, Phenyl, $C_7$-$C_{14}$ Alkaryl, $C_7$-$C_{14}$ Aralkyl, $C_2$-$C_{11}$ Alkoxyalkyl, eine Gruppe $-COR_{27}$, $-COOR_{28}$, $-CON(R_{29})(R_{30})$, $-CH_2-CH(R_{31})-OR_{32}$, $-SOR_{33}$ oder $-SO_2R_{33}$ ist, worin $R_{27}$ eine der für $R_{21}$ angegebenen Bedeutungen hat oder ein heterozyklischer Ring ist, $R_{28}$ eine der für $R_{18}$ angegebenen Bedeutungen hat, $R_{29}$ eine der für $R_{19}$ angegebenen Bedeutungen hat, $R_{30}$ eine der für $R_{20}$ angegebenen Bedeutungen hat, $R_{31}$ eine der für $R_{22}$ angegebenen Bedeutungen hat, $R_{32}$ eine der für $R_{23}$ angegebenen Bedeutungen hat und $R_{33}$ eine der für $R_{24}$ angegebenen Bedeutungen hat, oder $R_{26}$ eine der Gruppen

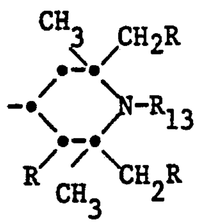 , 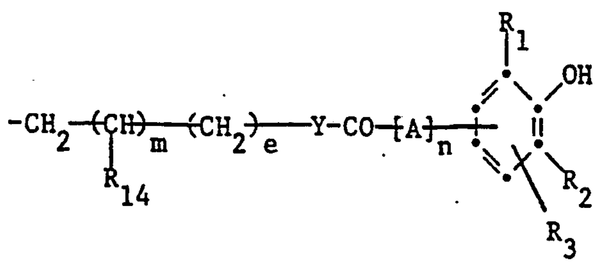

oder

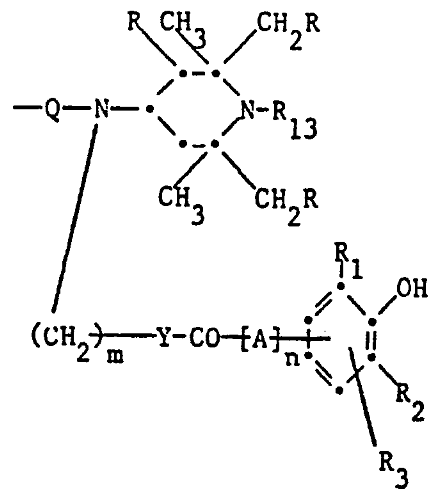

bedeutet,

Q eine Gruppe $-(-C_bH_{2b}-)-$, worin b eine Zahl 2 bis 12 bedeutet, $C_4$-$C_8$ Alkenylen, $C_5$-$C_{12}$ Cycloalkylen, Phenylen, Xylylen, Bitolylen, eine Gruppe $-CO-(-C_rH_{2r}-)-CO-$,

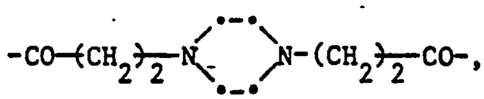, 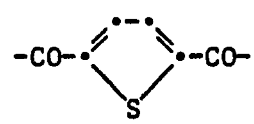, 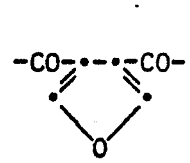,

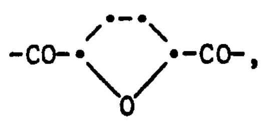, 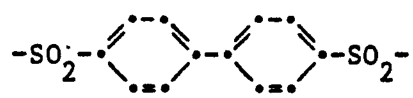,

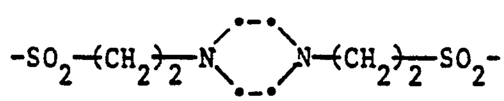

oder eine Gruppe 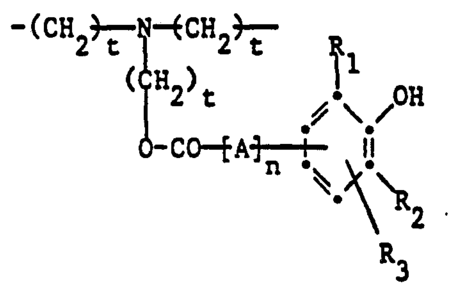

worin t eine Zahl 0 bis 5 ist, bedeutet, oder

$R_{13}$ eine Gruppe der Formel

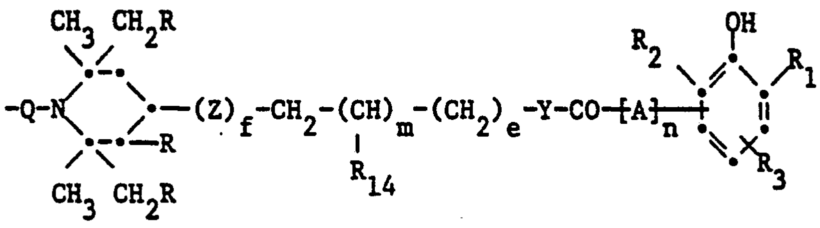

ist, und

$R_{14}$ Wasserstoff, $C_1$-$C_{18}$ Alkyl, $C_7$-$C_{23}$ Phenoxyalkyl, Phenyl, $C_7$-$C_{14}$-Aralkyl, $C_2$-$C_{11}$ Alkoxyalkyl oder eine Gruppe

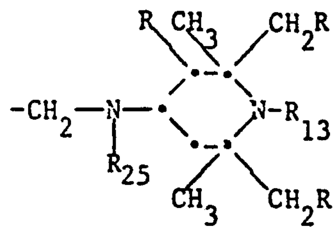 ist,

ist,

$R_{15}$ Wasserstoff, Methyl, Ethyl, Phenyl, Phenoxymethyl,

$R_{16}$ Wasserstoff, $-OR_{34}$, $-OCOR_{35}$, $-N(R_{36})-COR_{35}$, $-OSO_2R_{35}$, $-N(R_{36})-SO_2R_{35}$ ist, wobei $R_{34}$ Wasserstoff $C_1$-$C_{12}$ Alkyl, Allyl, Benzyl ist, $R_{35}$ Wasserstoff, $C_1$-$C_{12}$ Alkyl, $C_2$-$C_6$ Alkenyl, Chlormethyl, $C_5$-$C_8$ Cycloalkyl, $C_7$-$C_9$ Phenylalkyl, $C_7$-$C_{10}$ Alkylphenyl, Phenyl oder eine Gruppe

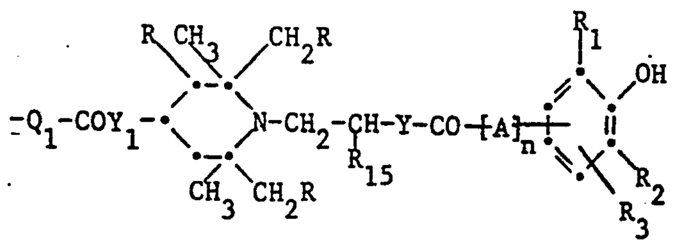

bedeutet, worin

$Y_1$ eine der für Y angegebenen Bedeutungen hat,

$Q_1$ eine Gruppe $-(-C_rH_{2r}-)-$, $C_4$-$C_8$ Alkenylen, $C_5$-$C_{12}$ Cycloalkylen, Phenylen, Xylylen, Bitolylen, eine Gruppe

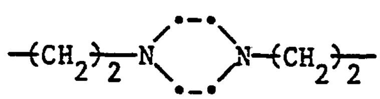 , 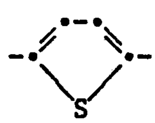 , 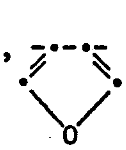 oder 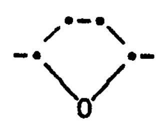 ist,

und $R_{36}$ Wasserstoff, $C_1$-$C_{12}$ Alkyl, $C_5$-$C_8$ Cycloalkyl oder Benzyl bedeutet und

$R_{17}$ Wasserstoff, Cyano, $-COOR_{18}$, $-CONH_2$, $-CON(R_{19})(R_{20})$ oder $-CH_2NHR_{37}$, worin $R_{37}$ eine Gruppe $-COR_{21}$, $-COOR_{18}$, $-CON(R_{19})(R_{20})$, $-CH_2-CH(R_{22})-OR_{23}$, $-SOR_{24}$, $-SO_2R_{24}$ ist, bedeutet, wobei die wiederholt erwähnten Reste und Symbole immer die erstmals angegebene Bedeutung haben.

2. Farbphotographisches Aufzeichnungsmaterial gemäss Anspruch 1, dadurch gekennzeichnet, dass es als Stabilisator mindestens eine Polyalkylpiperidinverbindung der Formel V enthält

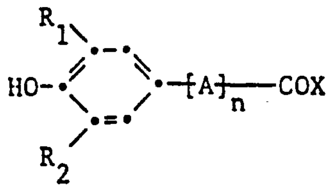 (V),

worin

n die Zahlen 0 oder 1 bedeutet,

$R_1$ Wasserstoff oder $C_1$-$C_4$ Alkyl ist,

$R_2$ $C_1$-$C_4$ Alkyl bedeutet,

A eine Gruppe $-CH_2-$, $-CH_2-CH(R_5)-$,

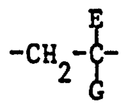 oder 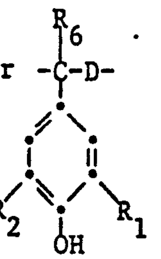

ist, worin $R_5$ und $R_6$ Wasserstoff oder Methyl sind, E Cyano, $COCH_3$ und $-COOCH_3$, G Wasserstoff, $C_1$-$C_{18}$ Alkyl, Allyl, Cyclohexyl, Benzyl oder eine Gruppe

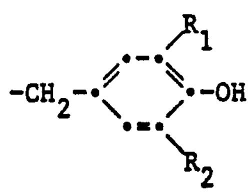

ist oder E und G zusammen eine Gruppe $-CO-(CH_2-)-_4$ bilden, D eine Gruppe $-(-C_rH_{2r}-)-$ ist, worin r die Zahlen 0 bis 6 bedeutet und

X eine Gruppe der Formel

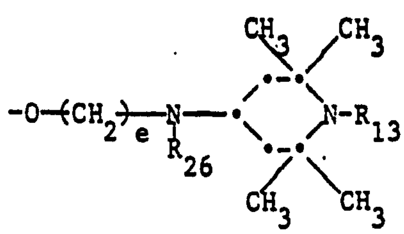

oder der Formel

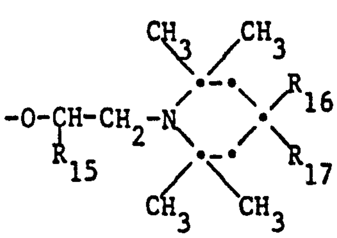

ist, worin

e die Zahlen 2 bis 3 bedeutet,

$R_{13}$ Hydroxy, Methyl, Allyl, Benzyl, 2-Hydroxyethyl, Acetyl, Acroyl oder eine Gruppe der Formel $-CON(R_{19})(R_{20})$, worin $R_{19}$ $C_1$-$C_8$ Alkyl, Cyclohexyl oder Phenyl und $R_{20}$ Wasserstoff oder $C_1$-$C_{12}$ Alkyl

sind, bedeutet,

$R_{26}$ Wasserstoff, $C_1$-$C_8$ Alkyl, eine Gruppe $-COR_{27}$, $COOR_{28}$, $-CON(R_{29})(R_{30})$, $-CH_2-CH(R_{31})-OR_{32}$ oder $-SO_2R_{33}$ ist, worin $R_{27}$ $C_1$-$C_{12}$ Alkyl, $C_2$-$C_4$ Alkenyl, Cyclohexyl, Benzyl, Phenyl, 2-(3,5-Di-tert.-butyl-4-hydroxyphenyl)-ethyl oder ein heterozyklischer Ring,

$R_{28}$ $C_1$-$C_8$ Alkyl, Allyl oder Cyclohexyl,

$R_{29}$ $C_1$-$C_{12}$ Alkyl, Cyclohexyl oder Phenyl,

$R_{30}$ Wasserstoff oder $C_1$-$C_{12}$ Alkyl sind oder

$R_{29}$ und $R_{30}$ zusammen mit dem N-Atom, an das sie gebunden sind, einen 6-gliedrigen heterocyclischen Ring bilden,

$R_{31}$ Wasserstoff, Methyl oder Phenyl und

$R_{32}$ Wasserstoff, $C_1$-$C_4$ Alkyl, eine Gruppe $-COR_{21}$ oder $-CON(R_{19})(R_{20})$ und

$R_{33}$ $C_1$-$C_{12}$ Alkyl, Phenyl oder $C_7$-$C_{10}$ Alkylphenyl sind,

$R_{15}$ Wasserstoff, Methyl oder Phenyl ist,

$R_{16}$ Wasserstoff, $-OR_{34}$, $-OCOR_{35}$ oder $-N(R_{36})-COR_{35}$ ist, wobei $R_{34}$ Wasserstoff, $C_1$-$C_8$ Alkyl, Allyl, Benzyl oder Phenyl ist, $R_{35}$ Wasserstoff, $C_1$-$C_8$ Alkyl, Allyl, Benzyl oder Phenyl bedeutet und $R_{36}$ Wasserstoff, $C_1$-$C_4$ Alkyl, Cyclohexyl, oder Benzyl ist und

$R_{17}$ Wasserstoff, Cyano, $-COOR_{18}$, $-CONH_2$ , $-CON(R_{19})(R_{20})$ oder $-CH_2NHR_{37}$, worin $R_{37}$ eine Gruppe $-COR_{21}$, $-COOR_{18}$, $-CON(R_{19})(R_{20})$, $-CH_2-CH(R_{22})-OR_{23}$ ist, bedeutet,

wobei die in diesem Anspruch wiederholt erwähnten Reste und Symbole immer die in diesem Anspruch erstmals angegebene Bedeutung haben.

3. Farbphotographisches Aufzeichnungsmaterial gemäss Anspruch 2, dadurch gekennzeichnet, dass es als Stabilisator mindestens eine Polyalkylpiperidinverbindung enthält, die der Formel V entspricht, worin

n die Zahlen 0 oder 1 bedeutet,

$R_1$ Wasserstoff, Methyl oder tert.-Butyl ist,

$R_2$ Methyl oder tert.-Butyl bedeutet,

A eine Gruppe $-CH_2-$ oder $-CH_2CH_2-$ ist, worin

X eine Gruppe der Formel

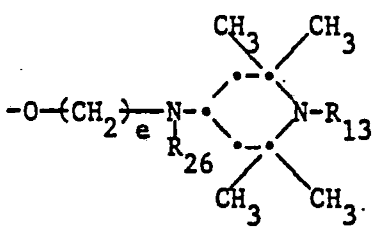 oder der Formel 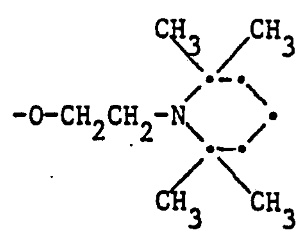

ist worin e die Zahlen 2 bis 3 bedeutet,

$R_{13}$ Methyl, Allyl, Benzyl, 2-Hydroxyethyl, Acetyl oder Acroyl ist,

$R_{26}$ Wasserstoff, $C_1$-$C_8$ Alkyl oder eine Gruppe der Formel $-COR_{27}$ oder $-SO_2R_{33}$ ist, worin $R_{27}$ $C_1$-$C_{12}$ Alkyl oder $C_2$-$C_4$ Alkenyl ist, und $R_{33}$ $C_1$-$C_4$ Alkyl, Phenyl oder p-Tolyl ist.

4. Farbphotographisches Aufzeichnungsmaterial gemäss Anspruch 1, dadurch gekennzeichnet, dass es als Stabilisator mindestens eine Polyalkylpiperidinverbindung der Formel I enthält, worin A eine der Gruppen $-CH_2-$, $-CH_2-CH(R_5)-$,

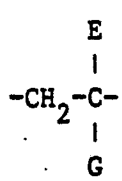 oder 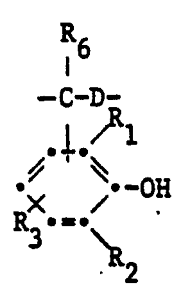 bedeutet.

5. Farbphotographisches Aufzeichnungsmaterial gemäss Anspruch 1, dadurch gekennzeichnet, dass es als Stabilisator mindestens eine Polyalkylpiperidinverbindung enthält, die der Formel VI entspricht

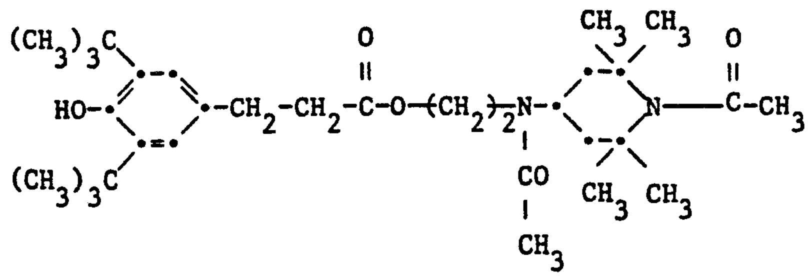

VI.

6. Farbphotographisches Aufzeichnungsmaterial gemäss Anspruch 1, dadurch gekennzeichnet, dass es als Stabilisator mindestens eine Polyalkylpiperidinverbindung enthält, die der Formel VII entspricht

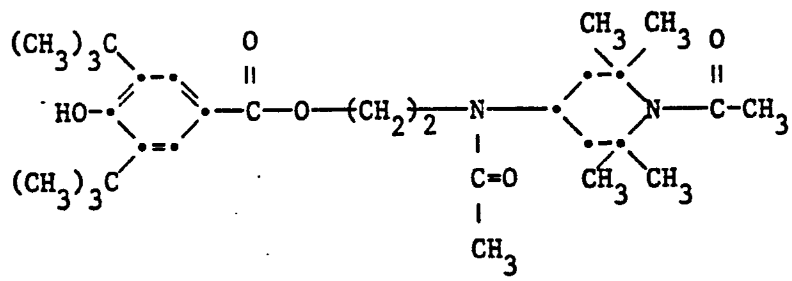

VII.

7. Farbphotographisches Aufzeichnungsmaterial gemäss Anspruch 1, dadurch gekennzeichnet, dass es als Stabilisator mindestens eine Polyalkylpiperidinverbindung enthält, die der Formel VIII entspricht

VIII.

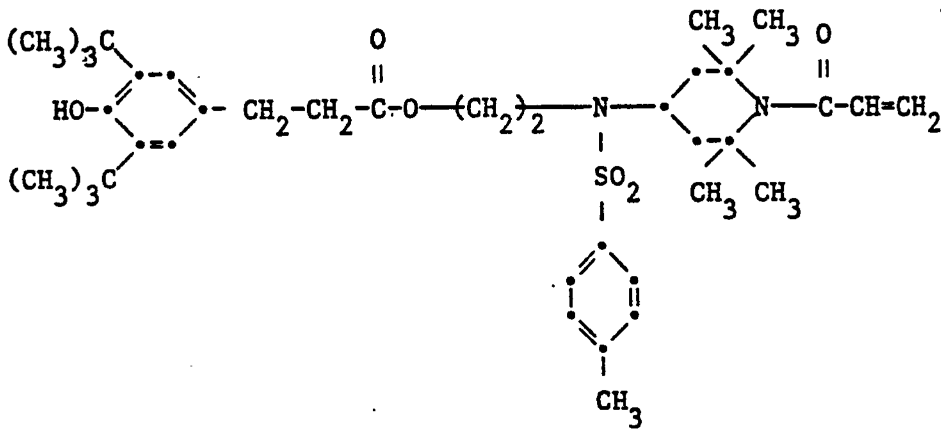

8. Farbphotographisches Aufzeichnungsmaterial gemäss Anspruch 1, dadurch gekennzeichnet, dass es als Stabilisator mindestens eine Polyalkylpiperidinverbindung enthält, die der Formel IX entspricht

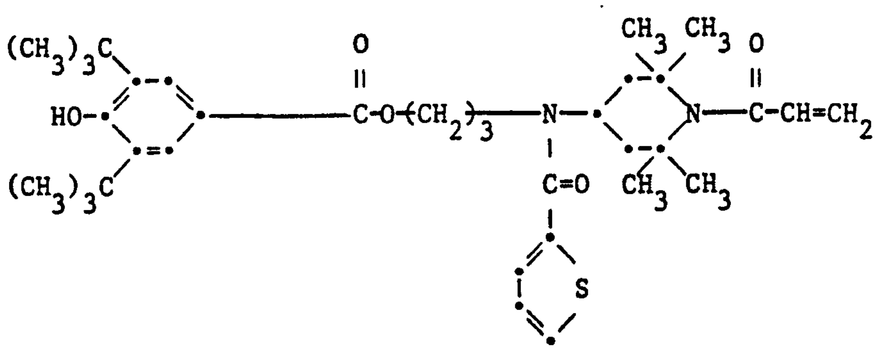

(IX).

9. Farbphotographisches Aufzeichnungsmaterial gemäss Anspruch 1, dadurch gekennzeichnet, dass es als Stabilisator mindestens eine Polyalkylpiperidinverbindung enthält, die der Formel X entspricht

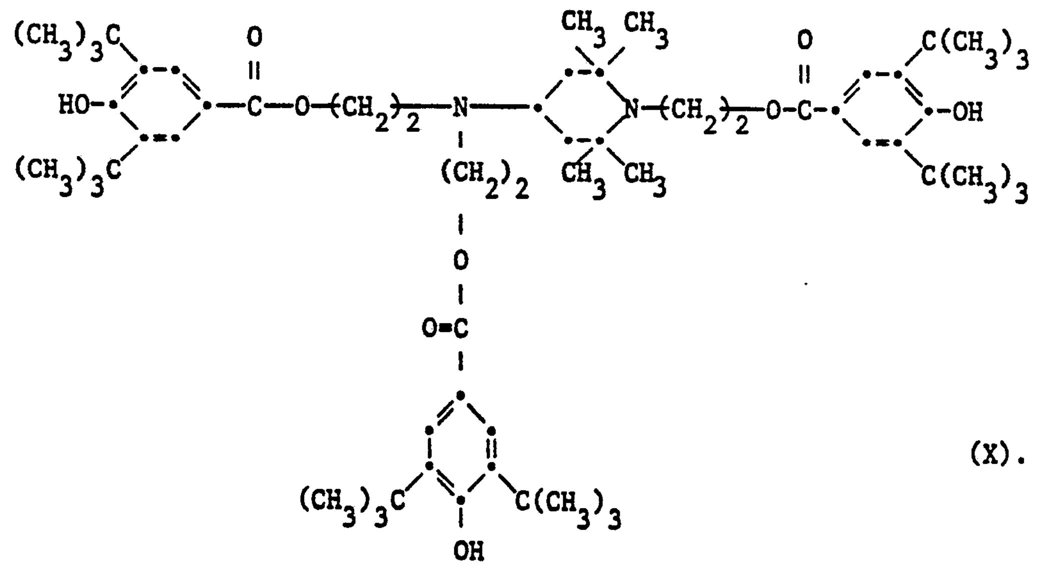

(X).

10. Farbphotographisches Aufzeichnungsmaterial gemäss Anspruch 1, dadurch gekennzeichnet, dass es die Stabilisatoren der Formel I in Kombination mit Blaugrün-, Purpur- und Gelbkupplern enthält.

11. Farbphotographisches Aufzeichnungsmaterial gemäss Anspruch 1, dadurch gekennzeichnet, dass es die Stabilisatoren der Formel I in Kombination mit Ultraviolettabsorbern enthält.

12. Farbphotographisches Aufzeichnungsmaterial gemäss Anspruch 11, dadurch gekennzeichnet, dass die Ultraviolettabsorber Verbindungen von Benzophenon-, Acrylnitril-, Thiazolidon-, Benztriazol-, Oxazol-, Thiazol- oder Imidazoltyp sind.

13. Farbphotographisches Aufzeichnungsmaterial gemäss Anspruch 1, dadurch gekennzeichnet, dass es die Stabilisatoren der Formel I in Kombination mit Blaugrün-, Purpur- und Gelbkupplern und mit UV-Absorbern in der gleichen Schicht enthält.

14. Farbphotographisches Aufzeichnungsmaterial gemäss Anspruch 1, dadurch gekennzeichnet, dass es 1 bis 2000 mg des Stabilisators der Formel I pro $m^2$ der Schicht enthält, in die er eingearbeitet ist.

15. Verfahren zur Herstellung von photographischen Farbbildern durch bildmässige Belichtung und Farbentwicklung eines farbphotographischen Aufzeichnungsmaterials gemäss Anspruch 1.

## Claims

1. A recording material for colour photography, which contains in at least one photosensitive silver halide emulsion layer, one intermediate layer and/or one protective layer, at least one polyalkylpiperidine compound as a stabiliser, wherein the polyalkylpiperidine compound is of the formula I

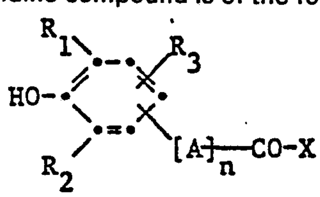

(I)

in which n is the number 0 or 1, $R_1$ is hydrogen, $C_1$-$C_8$-alkyl, $C_5$-$C_8$-cycloalkyl, phenyl, $C_7$-$C_{14}$-aralkyl, $C_7$-$C_{14}$-alkaryl or a group

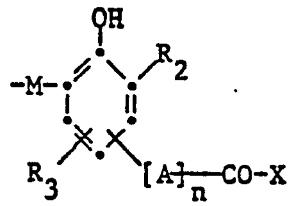

$R_2$ is $C_1$-$C_8$-alkyl, $C_5$-$C_8$-cycloalkyl, phenyl, $C_7$-$C_{14}$-aralkyl, $C_7$-$C_{14}$-alkaryl and $R_3$ is hydrogen or methyl, M is a direct bond, -S-, -S-S-, -S(O)-, -$S(O)_2$- or -$CH(R_4)$, in which $R_4$ is hydrogen or $C_1$-$C_8$-alkyl, A is a group -$CH_2$-, -$CH_2$-$CH(R_5)$-,

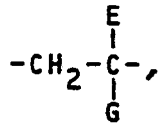

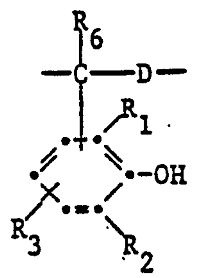

or

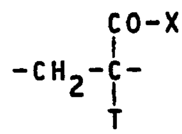

in which $R_5$ is hydrogen, methyl, ethyl, phenoxymethyl or phenyl, $R_6$ is hydrogen, $C_1$-$C_{12}$-alkyl, $C_5$-$C_8$ -cycloalkyl, phenyl or benzyl, E is -CN or a group -$COOR_7$, -$COR_8$, -$SO_2R_8$, -$P(O)(OR_9)_2$ or -CHO, in which $R_7$ is $C_1$-$C_4$-alkyl, $R_8$ is $C_1$-$C_{12}$-alkyl, $C_7$-$C_{14}$-alkaryl or phenyl and $R_9$ is $C_1$-$C_{18}$-alkyl, phenyl or allyl, G is hydrogen, $C_1$-$C_{18}$-alkyl, $C_3$-$C_{12}$-alkenyl, $C_3$-$C_4$-alkinyl, $C_3$-$C_{12}$-cycloalkyl, $C_6$-$C_{18}$-aralkyl-cycloalkyl, $C_6$-$C_{14}$-cycloalkylalkyl, $C_7$-$C_{14}$-aralkyl, $C_7$-$C_{14}$-alkaryl or phenyl or $C_1$-$C_{18}$-alkyl which is substituted by phenoxy, $C_7$-$C_{10}$-alkylphenoxy, benzyloxy, cyclohexyloxy, cyano, -$COOR_{10}$, -$OCOR_{11}$ or -$P(O)(OR_{12})_2$, in which $R_{10}$ is $C_1$-$C_{18}$-alkyl, $C_3$-$C_{12}$-cycloalkyl or a group of the formula II

$-(-C_aH_{2a}-)-CO-X$ (II)

in which a is a number from 1 to 6, $R_{11}$ is $C_1$-$C_{18}$-alkyl, $C_3$-$C_{12}$-cycloalkyl, phenyl, $C_7$-$C_9$-phenylalkyl or a group of the formula III

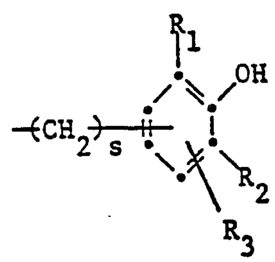

(III)

in which s is the number 0, 1 or 2, and $R_{12}$ is $C_1$-$C_8$-alkyl, allyl or phenyl, or G is $C_2$-$C_{18}$-alkyl which is interrupted by -O-, -S-, -SO- or -$SO_2$-, or is a group of the formula III, or, if E is a group -$COR_8$, G and $R_8$ together are tri- or tetramethylene which is unsubstituted or substituted by hydroxyl or oxo, D is a group $-(-C_rH_{2r}-)-$ or

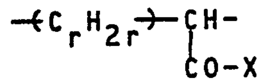

in which r is a number from 0 to 12, T is hydrogen, $C_1$-$C_{18}$-alkyl, $C_3$-$C_{12}$-alkenyl, $C_3$-$C_4$-alkynyl, $C_3$-$C_{12}$-cycloalkyl, $C_6$-$C_{18}$-alkyl-cycloalkyl, $C_6$-$C_{14}$-cycloalkylalkyl, $C_7$-$C_{14}$-aralkyl, $C_7$-$C_{14}$-alkaryl, phenyl, cyano or a group $-COR_8$, $-SO_2R_8$ or $-P(O)(OR_9)_2$, a group of the formula III, a group of the formula IV

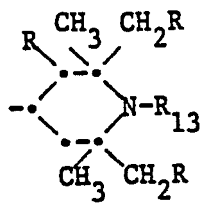

(IV)

or a group of the formula

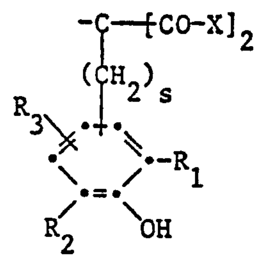

or T is $C_1$-$C_{18}$-alkyl which is interrupted by -O-, -S-, -SO- or $-SO_2-$, X is a group of the formula

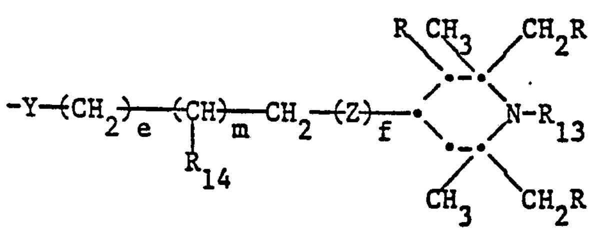

or of the formula

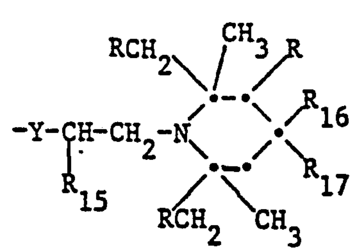

in which e is a number from 0 to 5, f and m independently of one another are each the number 0 or 1, R is hydrogen or methyl, $R_{13}$ is hydroxyl, $C_1$-$C_{12}$-alkyl, $C_3$-$C_6$-alkenyl methyl, $C_3$-$C_4$-alkynylmethyl, $C_7$-$C_{14}$-aralkyl or glycidyl, or $C_1$-$C_4$- alkyl which is substituted by halogen, cyano, $-COOR_{18}$ or $-CON(R_{19})(R_{20})$, or a group $-COR_{21}$,

$-COOR_{18}$ or $-CON(R_{19})-(R_{20})$, a group $-CH_2-CH(R_{22})-OR_{23}$, $-SOR_{24}$, $-SO_2R_{24}$, $-OR_{18}$ or $-OCOR_{21}$, in which $R_{18}$ is $C_1$-$C_{12}$-alkyl, allyl, cyclohexyl or benzyl, $R_{19}$ is $C_1$-$C_{12}$-alkyl, allyl, cyclohexyl, benzyl, phenyl or $C_7$-$C_{10}$-alkylphenyl and $R_{20}$ is hydrogen, $C_1$-$C_{12}$-alkyl, allyl, cyclohexyl or benzyl, or $R_{19}$ and $R_{20}$, together with the N atom to which they are bonded, are a 5-membered or 6-membered heterocyclic ring, $R_{21}$ is hydrogen, $C_1$-$C_{12}$-alkyl, $C_2$-$C_6$-alkenyl, chloromethyl, $C_5$-$C_8$-cycloalkyl, $C_7$-$C_{14}$-aralkyl, phenyl or $C_7$-$C_{10}$ -alkylphenyl, or phenyl, phenylmethyl or phenylethyl which is substituted by 1 or 2 $C_1$-$C_4$-alkyl groups and 1 hydroxyl group, $R_{22}$ is hydrogen, $C_1$-$C_4$-alkyl, $C_3$-$C_4$-alkoxyalkyl, phenyl or phenoxymethyl, $R_{23}$ is hydrogen, $C_1$-$C_{12}$-alkyl or a group $-COR_{21}$ or $-CON(R_{19})(R_{20})$ and $R_{24}$ is $C_1$-$C_{12}$-alkyl, phenyl or $C_7$-$C_{10}$-alkylphenyl, Y is -O- or $-N(R_{25})-$, in which $R_{25}$ is hydrogen, $C_1$-$C_{18}$-alkyl, $C_3$-$C_{12}$-alkenyl, $C_3$-$C_{12}$-cycloalkyl, phenyl, $C_7$-$C_{14}$-aralkyl, $C_7$-$C_{14}$-alkaryl, $C_2$-$C_{11}$-alkoxyalkyl or a group of the formula IV, Z is -O- or $-N(R_{26})-$, in which $R_{26}$ is hydrogen, $C_1$-$C_{18}$-alkyl, $C_3$-$C_{12}$-alkenyl, $C_3$-$C_{12}$-cycloalkyl, phenyl, $C_7$-$C_{14}$-alkaryl, $C_7$-$C_{14}$-aralkyl, $C_2$-$C_{11}$-alkoxyalkyl or a group $-COR_{27}$, $-COOR_{28}$, $-CON(R_{29})(R_{30})$, $-CH_2-CH(R_{31})-OR_{32}$, $-SOR_{33}$ or $-SO_2R_{33}$ , in which $R_{27}$ has one of the meanings given for $R_{21}$ or is a heterocyclic ring, $R_{28}$ has one of the meanings given for $R_{18}$, $R_{29}$ has one of the meanings given for $R_{19}$, $R_{30}$ has one of the meanings given for $R_{20}$, $R_{31}$ has one of the meanings given for $R_{22}$, $R_{32}$ has one of the meanings given for $R_{23}$ and $R_{33}$ has one of the meanings given for $R_{24}$, or $R_{26}$ is one of the groups

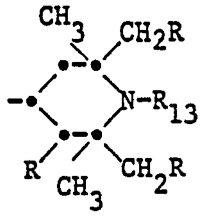
,

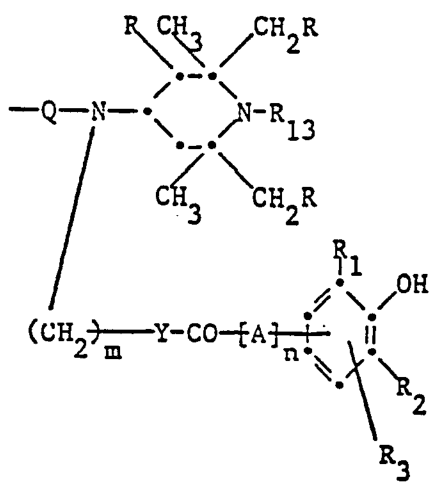

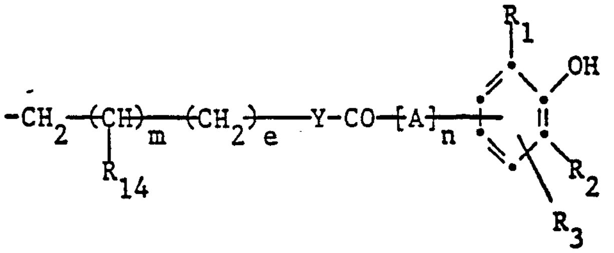
or

Q is a group $-(C_bH_{2b})-$, in which b is a number from 2 to 12, $C_4$-$C_8$-alkenylene, $C_5$-$C_{12}$-cycloalkylene, phenylene, xylylene bitolylene, a group $-CO-(C_rH_{2r})-CO-$,

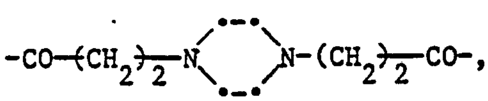
,
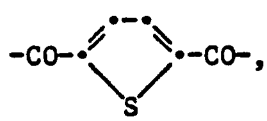
,
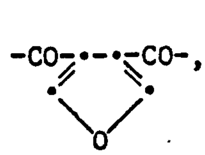
,

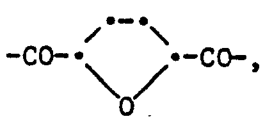
,
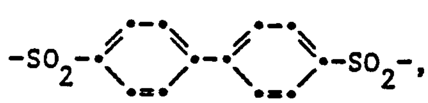
,

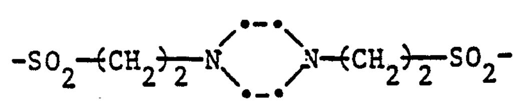

or a group

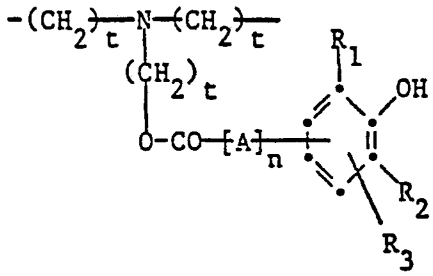

in which t is a number from 0 to 5, or $R_{13}$ is a group of the formula

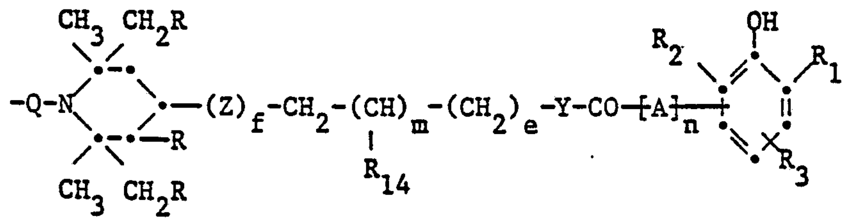

and $R_{14}$ is hydrogen, $C_1$-$C_{18}$-alkyl, $C_7$-$C_{23}$-phenoxyalkyl, phenyl, $C_7$-$C_{14}$-aralkyl, $C_2$-$C_{11}$-alkoxyalkyl or a group

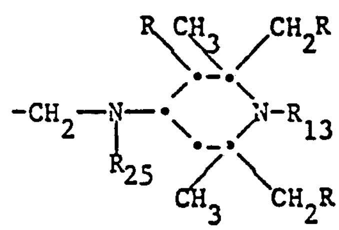

$R_{15}$ is hydrogen, methyl, ethyl, phenyl or phenoxymethyl, $R_{16}$ is hydrogen, $-OR_{34}$, $-OCOR_{35}$, $-N(R_{36})-COR_{35}$, $-OSO_2R_{35}$ or $-N(R_{36})-SO_2R_{35}$, in which $R_{34}$ is hydrogen, $C_1$-$C_{12}$-alkyl, allyl, or benzyl, $R_{35}$ is hydrogen, $C_1$-$C_{12}$-alkyl, $C_2$-$C_6$-alkenyl chloromethyl, $C_5$-$C_8$-cycloalkyl, $C_7$-$C_9$-phenylalkyl, $C_7$-$C_{10}$-alkylphenyl, phenyl or a group

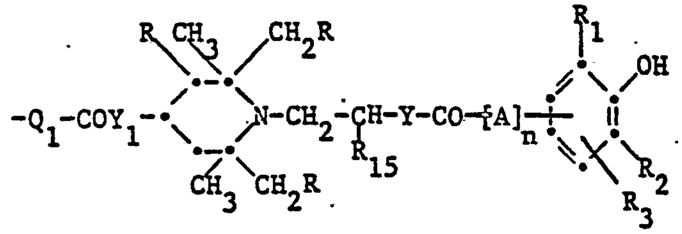

in which $Y_1$ has one of the meanings given for Y, $Q_1$ is a group $-(C_rH_{2r})-$, $C_4$-$C_8$-alkenylene, $C_5$-$C_{12}$-cycloalkylene, phenylene, xylylene, bitolylene or a group

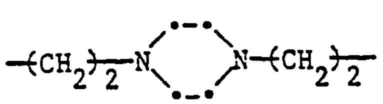 , 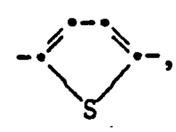 , 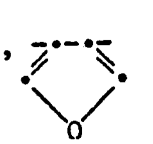 or 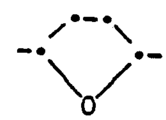

and $R_{36}$ is hydrogen, $C_1$-$C_{12}$-alkyl, $C_5$-$C_8$-cycloalkyl or benzyl, and $R_{17}$ is hydrogen, cyano, $-COOR_{18}$, $-CONH_2$, $-CON(R_{19})(R_{20})$ or $-CH_2NHR_{37}$, in which $R_{37}$ is a group $-COR_{21}$, $-COOR_{18}$, $-CON(R_{19})(R_{20})$, $-CH_2-CH(R_{22})-OR_{23}$, $-SOR_{24}$ or $-SO_2R_{24}$, the radicals and symbols mentioned repeatedly always being as first defined.

2. A recording material for colour photography according to Claim 1, which contains, as the stabiliser, at least one polyalkylpiperidine compound of the formula V

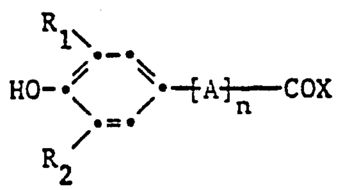

(V)

in which n is the number 0 or 1, $R_1$ is hydrogen or $C_1$-$C_4$-alkyl, $R_2$ is $C_1$-$C_4$-alkyl, A is a group $-CH_2-$, $-CH_2-CH(R_5)$,

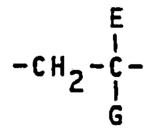

or

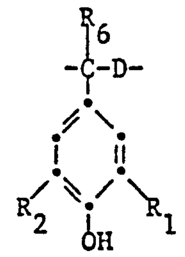

in which $R_5$ and $R_6$ are hydrogen or methyl, E is cyano, $-COCH_3$ or $-COOCH_3$, G is hydrogen, $C_1$-$C_{18}$-alkyl, allyl, cyclohexyl, benzyl or a group

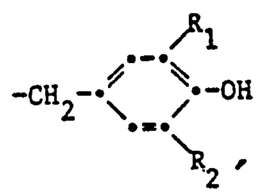

,

or E and G together are a group $-CO-(CH_2)_4$, D is a group $-(C_rH_{2r})-$, in which r is a number from 0 to 6, and X is a group of the formula

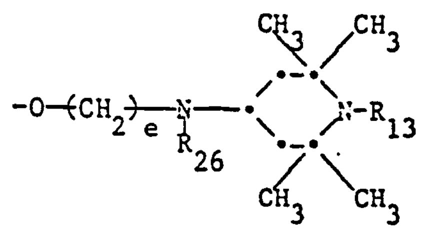

or of the formula

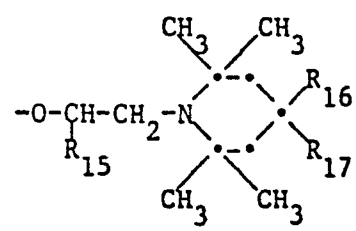

in which e is a number from 2 to 3, $R_{13}$ is hydroxyl, methyl, allyl, benzyl, 2-hydroxyethyl, acetyl, acroyl or a group of the the formula $-CON(R_{19})(R_{20})$, in which $R_{19}$ is $C_1$-$C_8$-alkyl, cyclohexyl or phenyl and $R_{20}$ is hydrogen or $C_1$-$C_{12}$-alkyl, $R_{26}$ is hydrogen, $C_1$-$C_8$-alkyl or a group $-COR_{27}$, $-COOR_{28}$, $-CON(R_{29})(R_{30})$, $-CH_2-CH(R_{31})-OR_{32}$ or $-SO_2R_{33}$, in which $R_{27}$ is $C_1$-$C_{12}$-alkyl, $C_2$-$C_4$-alkenyl, cyclohexyl, benzyl, phenyl, 2-(3,5-di-tert.-butyl-4-hydroxyphenyl)-ethyl or a heterocyclic ring, $R_{28}$ is $C_1$-$C_8$-alkyl, allyl or cyclohexyl, $R_{29}$ is $C_1$-$C_{12}$-alkyl, cyclohexyl or phenyl and $R_{30}$ is hydrogen or $C_1$-$C_{12}$-alkyl, or $R_{29}$ and $R_{30}$ , together with the N atom to which they are bonded, are a 6-membered heterocyclic ring, $R_{31}$ is hydrogen, methyl or phenyl and $R_{32}$ is hydrogen, $C_1$-$C_4$-alkyl or a group $-COR_{21}$ or $-CON(R_{19}(R_{20})$ and $R_{33}$ is $C_1$-$C_{12}$-alkyl, phenyl or $C_7$-$C_{10}$-alkylphenyl, $R_{15}$ is hydrogen, methyl or phenyl, $R_{16}$ is hydrogen, $-OR_{34}$, $-OCOR_{35}$, or $-N(R_{36})-COR_{35}$, in which $R_{34}$ is hydrogen, $C_1$-$C_8$-alkyl, allyl, benzyl or phenyl, $R_{35}$ is hydrogen, $C_1$-$C_8$-alkyl, allyl, benzyl or phenyl and $R_{36}$ is hydrogen, $C_1$-$C_4$ -alkyl, cyclohexyl or benzyl, and $R_{17}$ is hydrogen, cyano, $-COOR_{18}$, $-CONH_2$, $-CON(R_{19})(R_{20})$ or $-CH_2NHR_{37}$, in which $R_{37}$ is a group $-COR_{21}$, $-COOR_{18}$, $-CON(R_{19})(R_{20})$ or $-CH_2-CH(R_{22})-OR_{23}$ , the radicals and symbols repeatedly mentioned in this claim always being as first defined in this claim.

3. A recording material for colour photography according to Claim 1, which contains, as the stabiliser, at least one polyalkylpiperidine compound which is of the formula V in which n is the number 0 or 1, $R_1$ is hydrogen, methyl or tert.butyl, $R_2$ is methyl or tert.-butyl, A is a group $-CH_2-$ or $-CH_2CH_2-$, in which X is a group of the formula

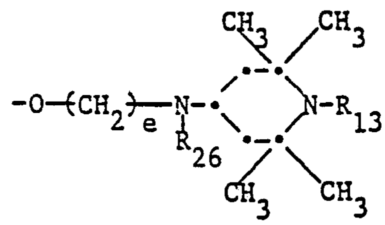

or of the formula

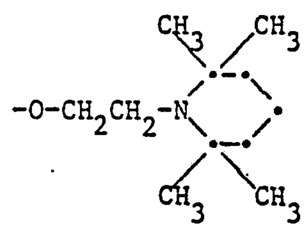

in which e is a number from 2 to 3, $R_{13}$ is methyl, allyl, benzyl, 2-hydroxyethyl, acetyl or acroyl and $R_{26}$ is hydrogen, $C_1$-$C_8$-alkyl or a group of the formula $-COR_{27}$ or $-SO_2R_{33}$, in which $R_{27}$ is $C_1$-$C_{12}$-alkyl or $C_2$-$C_4$-alkenyl and $R_{33}$ is $C_1$-$C_4$-alkyl, phenyl or p-tolyl.

4. A recording material for colour photography according to Claim 1, which contains, as the stabiliser, at least one polyalkylpiperidine compound of the formula I in which A is one of the groups $-CH_2-$, $-CH_2-CH(R_5)-$,

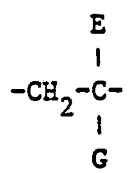

or

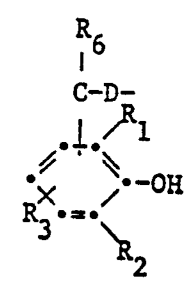

5. A recording material for colour photography according to Claim 1, which contains, as the stabiliser, at least one polyalkylpiperidine compound which is of the formula VI

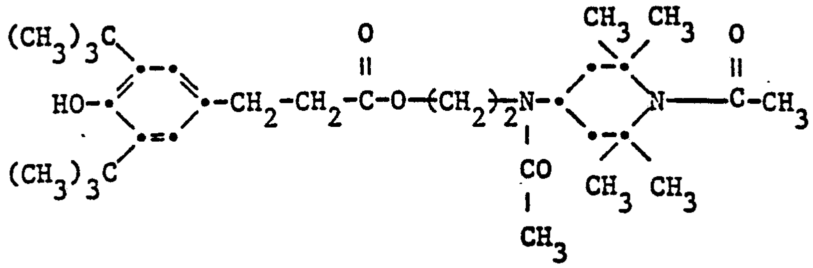

VI.

6. A recording material for colour photography according to Claim 1, which contains, as the stabiliser, at least one polyalkylpiperidine compound which is of the formula VII

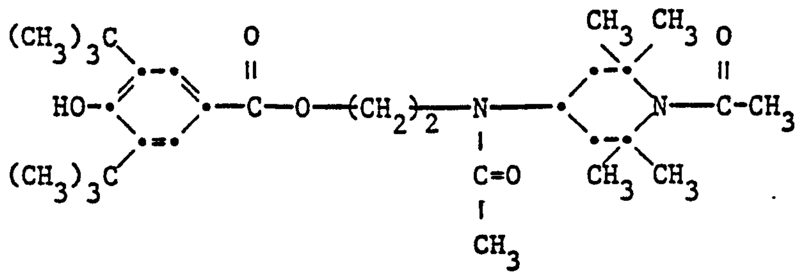

VII.

7. A recording material for colour photography according to Claim 1, which contains, as the stabiliser, at least one polyalkylpiperidine compound which is of the formula VIII

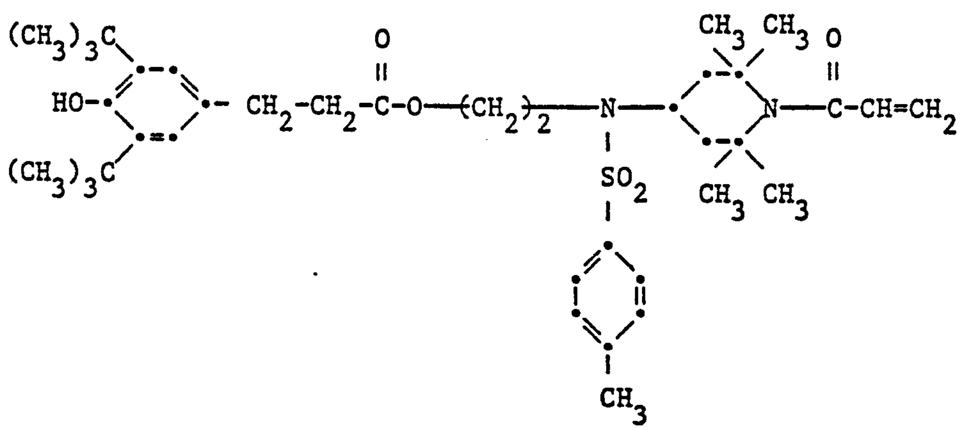

VIII.

8. A recording material for colour photography according to Claim 1, which contains, as the stabiliser, at least one polyalkylpiperidine compound which is of the formula IX

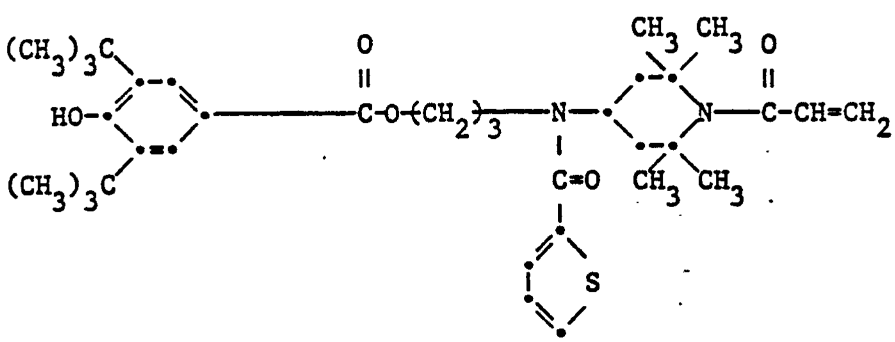

IX.

9. A recording material for colour photography according to Claim 1, which contains, as the stabiliser, at least one polyalkylpiperidine compound which is of the formula X

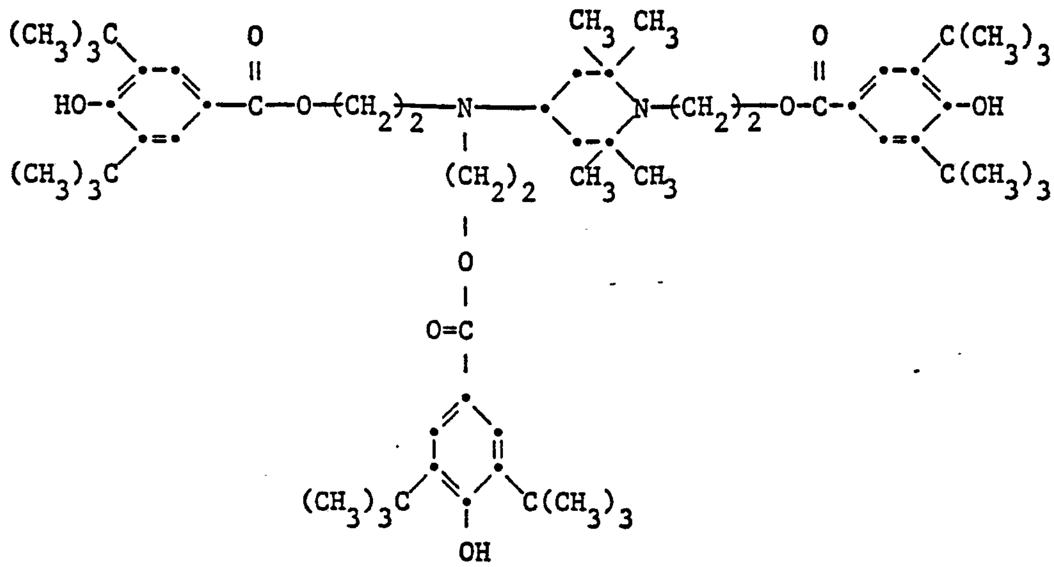

X.

10. A recording material for colour photography according to Claim 1, which contains a stabiliser of the formula I in combination with a cyan, magenta or yellow coupler.

11. A recording material for colour photography according to Claim 7, which contains a stabiliser of the formula I in combination with ultraviolet absorbers.

12. A recording material for colour photography according to Claim 11, wherein the ultraviolet absorbers are compounds of the benzophenone, acrylonitrile, thiazolidone, benzotriazole, oxazole, thiazole or imidazole type.

13. A recording material for colour photography according to Claim 1, which contains a stabiliser of the formula I in combination with a cyan, magenta or yellow coupler and with a UV absorber in the same layer.

14. A recording material for colour photography according to Claim 1, which contains 1 to 2,000 mg of the stabiliser of the formula I per $m^2$ of the layer in which it is incorporated.

15. A process for the preparation of photographic colour-forming agents by imagewise exposure and colour development of a recording material for colour photography according to Claim 1.

**Revendications**

1. Matière pour enregistrement photographique en couleurs qui contient, comme stabilisant, au moins un composé polyalkyl-pipéridinique dans au moins une couche d'émulsion d'halogénure d'argent photosensible, une couche intermédiaire et/ou une couche de protection, matière caractérisée en ce que le composé polyalkyl-pipéridinique répond à la formule I:

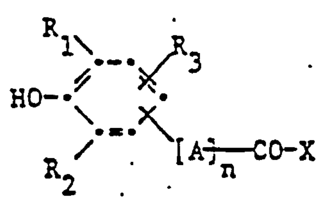

(I)

dans laquelle:

n représente le nombre 0 ou le nombre 1,

$R_1$ représente l'hydrogène, un alkyle en $C_1$-$C_8$, un cycloalkyle en $C_5$-$C_8$, un phényle, un aralkyle en $C_7$-$C_{14}$, un alkylaryle en $C_7$-$C_{14}$ ou un radical:

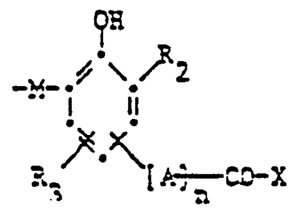

$R_2$ représente un alkyle en $C_1$-$C_8$, un cycloakyle en $C_5$-$C_8$, un phényle, un aralkyle en $C_7$-$C_{14}$ ou un alkylaryle en $C_7$-$C_{14}$,

$R_3$ représente l'hydrogène ou un méthyle,

M représente une liaison directe ou un pont -S-, -S-S-, -S(O)-, -S(O)$_2$- ou -CH($R_4$)-, le symbole $R_4$ présent dans ce dernier représentant l'hydrogène ou un alkyle en $C_1$-$C_8$,

A représente l'un des radicaux -$CH_2$-, -$CH_2$-CH($R_5$)-,

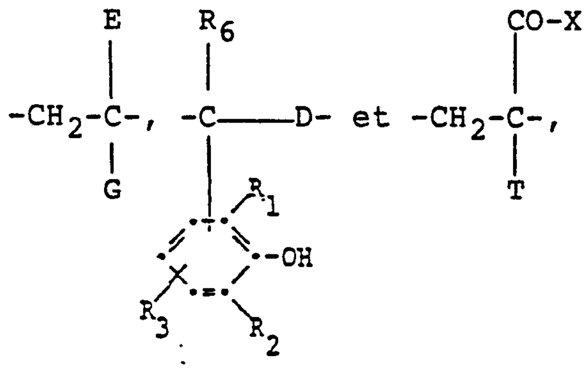

où

R5 représente l'hydrogène ou radical méthyle, éthyle, phénoxyméthyle ou phényle,

$R_6$ représente l'hydrogène, un alkyle en $C_1$-$C_{12}$, un cycloalkyle en $C_5$-$C_8$, un phényle ou un benzyle,

E représente un radical -CN, -COOR$_7$, -COR$_8$, -SO$_2$R$_8$, -P(O)(OR$_9$)$_2$ ou -CHO, le symbole $R_7$ désignant un alkyle en $C_1$-$C_4$, $R_8$ un alkyle en $C_1$-$C_{12}$, un alkylaryle en $C_7$-$C_{14}$ ou un phényle, et $R_9$ un alkyle en $C_1$-$C_{18}$, un phényle ou un allyle,

G représente l'hydrogène, un alkyle en $C_1$-$C_{18}$, un alcényle en $C_3$-$C_{12}$, un alcynyle en $C_3$ ou $C_4$, un cycloalkyle en $C_3$-$C_{12}$, un alkylcycloalkyle en $C_6$-$C_{18}$, un cycloalkylalkyle en $C_6$-$C_{14}$, un aralkyle en $C_7$-$C_{14}$, un alkylaryle en $C_7$-$C_{14}$, un phényle ou un alkyle en $C_1$-$C_{18}$ qui porte un phénoxy, un alkylphénoxy en $C_7$-$C_{10}$, un benzyloxy, un cyclohexyloxy, un cyano ou un radical -COOR$_{10}$, -OCOR$_{11}$ ou -P(O)(OR$_{12}$)$_2$, le symbole $R_{10}$ représentant un alkyle en $C_1$-$C_{18}$, un cycloalkyle en $C_3$-$C_{12}$ ou un radical répondant à la formule II:

iH$_{2a}$)-O-X (II)

dans laquelle a représente un nombre de 1 à 6,

$R_{11}$ représente un alkyle en $C_1$-$C_{18}$, un cycloalkyle en $C_3$-$C_{12}$, un phényle, un phénylalkyle en $C_7$-$C_9$ ou un radical répondant à la formule III:

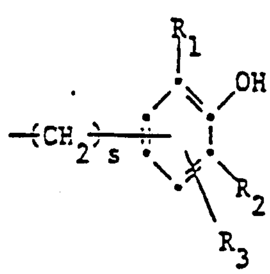

(III)

dans laquelle s désigne un nombre égal à 0, à 1 ou à 2,

$R_{12}$ représente un alkyle en $C_1$-$C_8$, un allyle ou un phényle,

ou

G représente un alkyle en $C_2$-$C_{18}$ interrompu par -O-, -S-, -SO- ou -SO$_2$- ou un radical de formule III, ou encore, lorsque E représente un radical -COR$_8$, G et $R_8$ forment ensemble un radical triméthylène ou tétraméthylène non substitué ou porteur d'un hydroxy ou d'un oxo,

D représente un radical —$(C_rH_{2r})$— ou un radical

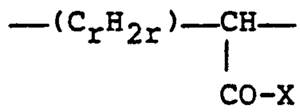

dans lesquels r désigne un nombre de 0 à 12,

T représente l'hydrogène, un alkyle en $C_1$-$C_{18}$, un alcényle en $C_3$-$C_{12}$, un alkynyle en $C_3$ ou $C_4$, un cycloalkyle en $C_3$-$C_{12}$, un alkylcycloalkyle en $C_6$-$C_{18}$, un cycloalkylalkyle en $C_6$-$C_{14}$, un aralkyle en $C_7$-$C_{14}$, un alkylaryle en $C_7$-$C_{14}$, un phényle, un cyano, un radical $-COR_8$, $-SO_2R_8$ ou $-P(O)(OR_9)_2$, un radical de formule III, un radical de formule IV:

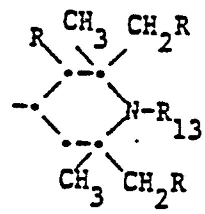

(IV)

ou un radical de formule:

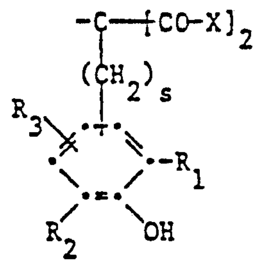

ou encore T représente un alkyle en $C_1$-$C_{18}$ interrompu par -O-, -S-, -SO- ou $-SO_2-$,

X représente un radical répondant à la formule:

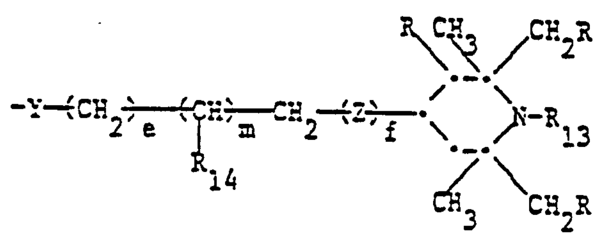

ou à la formule:

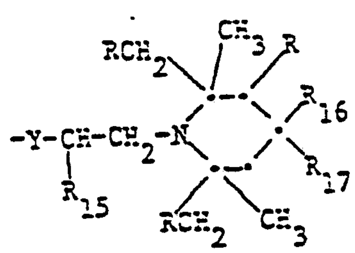

dans lesquelles e représente un nombre de 0 à 5 tandis que f et m représentent chacun, indépendamment l'un de l'autre, le nombre 0 ou le nombre 1,

R représente l'hydrogène ou un méthyle,

$R_{13}$ représente un hydroxy, un alkyle en $C_1$-$C_{12}$, un alcénylméthyle en $C_3$-$C_6$, un alcynylméthyle en

$C_3$ ou $C_4$, un aralkyle en $C_7$-$C_{14}$, un glycidyle, un alkyle en $C_1$-$C_4$ porteur d'un halogène, d'un cyano, d'un radical $-COOR_{18}$ ou d'un radical $-CON(R_{19})(R_{20})$, un radical $-COR_{21}$, $-COOR_{18}$ ou $-CON(R_{19})(R_{20})$, un radical $-CH_2-CH(R_{22})-OR_{23}$, $-SOR_{24}$, $-SO_2R_{24}$, $-OR_{18}$ ou $-OCOR_{21}$, radicaux dans lesquels $R_{18}$ représente un alkyle en $C_1$-$C_{12}$, un allyle, un cyclohexyle ou un benzyle, $R_{19}$ un alkyle en $C_1$-$C_{12}$, un allyle, un cyclohexyle, un benzyle, un phényle ou un alkylphényle en $C_7$-$C_{10}$, $R_{20}$ l'hydrogène, un alkyle en $C_1$-$C_{12}$, un allyle, un cyclohexyle ou un benzyle, ou encore $R_{19}$ et $R_{20}$ forment ensemble, et avec l'atome d'azote auquel ils sont liés, un noyau hétérocyclique à 5 ou 6 maillons, $R_{21}$ représente l'hydrogène, un alkyle en $C_1$-$C_{12}$, un alcényle en $C_2$-$C_6$, un chlorométhyle, un cycloalkyle en $C_5$-$C_8$, un aralkyle en $C_7$-$C_{14}$, un phényle, un alkylphényle en $C_7$-$C_{10}$ ou un radical phényle, phénylméthyle ou phényléthyle porteur d'un ou de deux alkyles en $C_1$-$C_4$ et d'un hydroxy, $R_{22}$ l'hydrogène, un alkyle en $C_1$-$C_4$, un alcoxyalkyle en $C_3$-$C_4$, un phényle ou un phénoxyméthyle, $R_{23}$ l'hydrogène, un alkyle en $C_1$-$C_{12}$, un radical $-COR_{21}$ ou un radical $-CON(R_{19})(R_{20})$, et $R_{24}$ un alkyle en $C_1$-$C_{12}$, un phényle ou un alkylphényle en $C_7$-$C_{10}$,

Y représente -O- ou un radical $-N(R_{25})$- dans lequel $R_{25}$ représente l'hydrogène, un alkyle en $C_1$-$C_{18}$, un alcényle en $C_3$-$C_{12}$, un cycloalkyle en $C_3$-$C_{12}$, un phényle, un aralkyle en $C_7$-$C_{14}$, un alkylaryle en $C_7$-$C_{14}$, un alcoxyalkyle en $C_2$-$C_{11}$ ou un radical de formule IV,

Z représente -O- ou un radical $-N(R_{26})$- dans lequel $R_{26}$ représente l'hydrogène, un alkyle en $C_1$-$C_{18}$, un alcényle en $C_3$-$C_{12}$, un cycloalkyle en $C_3$-$C_{12}$, un phényle, un alkylaryle en $C_7$-$C_{14}$, un aralkyle en $C_7$-$C_{14}$, un alcoxyalkyle en $C_2$-$C_{11}$ ou l'un des radicaux $-COR_{27}$, $-COOR_{28}$, $-CON(R_{29})(R_{30})$, $-CH_2-CH(R_{31})-OR_{32}$, $-SOR_{33}$ et $-SO_2R_{33}$, dans lesquels $R_{27}$ a l'une des significations qui ont été données pour $R_{21}$ ou représente un noyau hétérocyclique, $R_{28}$ a l'une des significations qui ont été données pour $R_{18}$, $R_{29}$ l'une des significations qui ont été données pour $R_{19}$, $R_{30}$ l'une des significations qui ont été données pour $R_{20}$, $R_{31}$ l'une des significations qui ont été données pour $R_{22}$, $R_{32}$ l'une des significations qui ont été données pour $R_{23}$, et $R_{33}$ l'une des significations qui ont été données pour $R_{24}$, ou encore $R_{26}$ représente un radical répondant à l'une des formules suivantes:

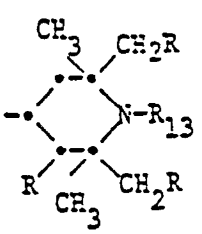
,
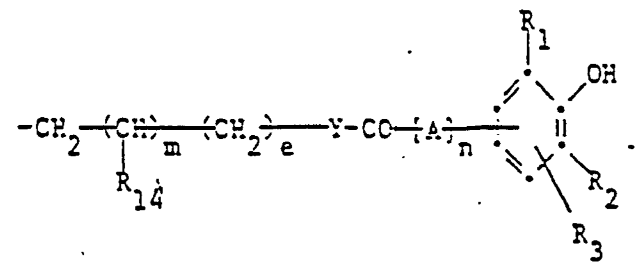

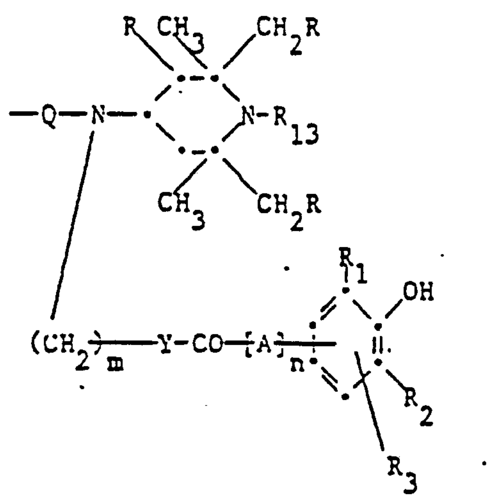

Q représente un radical $-(C_bH_{2b})$- dans lequel b désigne un nombre de 2 à 12, un alcénylène en $C_4$-$C_8$, un cycloalkyléne en $C_5$-$C_{12}$, un phénylène, un xylylène, un bitolylène, un radical $-CO-(-C_rH_2r-)-CO-$,

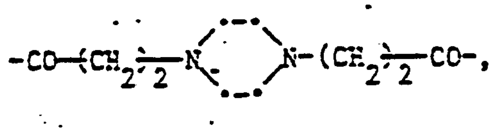, 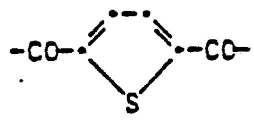, 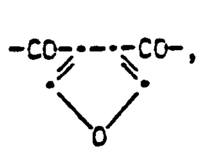,

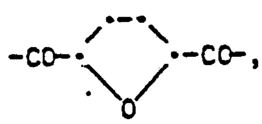, 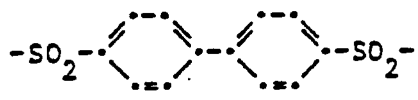

ou

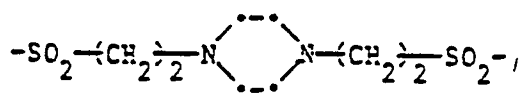

ou un radical répondant à la formule suivante:

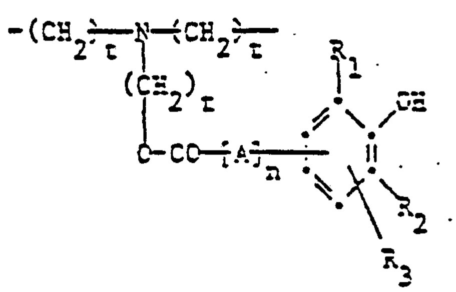

dans laquelle t désigne un nombre de 0 à 5,

$R_{13}$ représente un radical de formule:

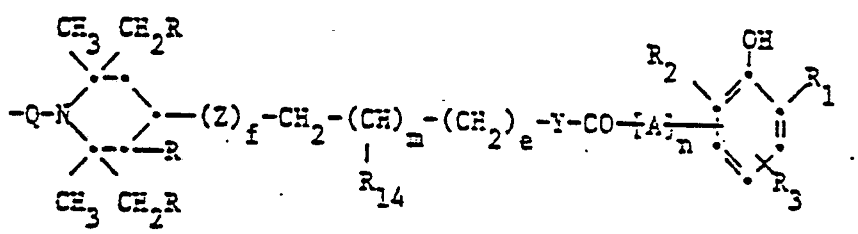

$R_{14}$ représente l'hydrogène un alkyle en $C_1$-$C_{18}$, un phénoxyalkyle en $C_7$-$C_{23}$, un phényle, un aralkyle en $C_7$-$C_{14}$, un alcoxyalkyle en $C_2$-$C_{11}$ ou un radical:

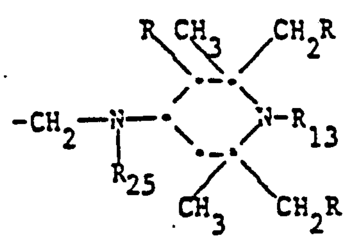

$R_{15}$ représente l'hydrogène ou un radical méthyle, éthyle, phényle ou phénoxyméthyle,

$R_{16}$ représente l'hydrogène ou un radical $-OR_{34}$, $-OCOR_{35}$, $-N(R_{36})-COR_{35}$, $-OSO_2R_{35}$ ou $-N(R_{36})-SO_2R_{35}$, le symbole $R_{34}$ représentent l'hydrogène, un alkyle en $C_1$-$C_{12}$, un allyle ou un benzyle, $R_{35}$ l'hydrogène, un alkyle en $C_1$-$C_{12}$, un alcényle en $C_2$-$C_6$, un chlorométhyle, un cycloalkyle

en $C_5$-$C_8$, un phénylalkyle en $C_7$-$C_9$, un alkylphényle en $C_7$-$C_{10}$, un phényle ou un radical répondant à la formule suivante:

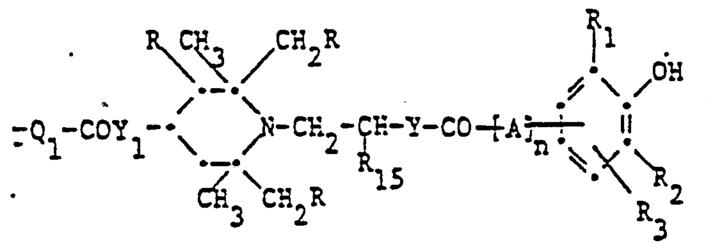

dans laquelle $Y_1$ a l'une des significations qui ont été données pour Y et $Q_1$ représente un radical $-(C_rH_{2r})-$, un alcénylène en $C_4$-$C_8$, un cycloalkylène en $C_5$-$C_{12}$, un phénylène, un xylylène, un bitolylène ou un radical:

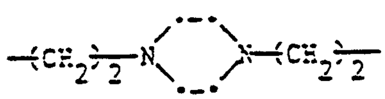 , 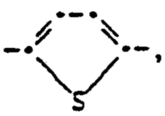 , 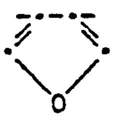 ou 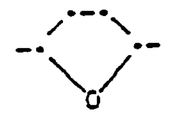 ,

et $R_{36}$ l'hydrogène, un alkyle en $C_1$-$C_{12}$, un cycloalkyle en $C_5$-$C_8$ ou un benzyle,

et

$R_{17}$ représente l'hydrogène, un cyano, ou un radical $-COOR_{18}$, $-CONH_2$, $-CON(R_{19})(R_{20})$ ou $-CH_2NHR_{37}$, le symbole $R_{37}$ représentant un radical $-COR_{21}$, $-COOR_{18}$, $-CON(R_{19})(R_{20})$, $-CH_2-CH(R_{22})-OR_{23}$, $-SOR_{24}$ ou $-SO_2R_{24}$,

les radicaux et symboles mentionnés plusieurs fois ayant toujours la signification qui leur a été attribuée la première fois qu'ils ont été rencontrés.

2. Matière pour enregistrement photographique en couleurs selon la revendication 1, caractérisée en ce quelle contient, comme stabilisant, au moins un composé polyalkyl-pipéridinique répondant à la formule V:

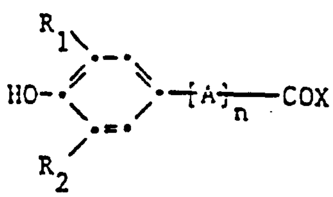 (V),

dans laquelle:

n représente le nombre 0 ou le nombre 1,

$R_1$ représente l'hydrogène ou un alkyle en $C_1$-$C_4$,

$R_2$ représente un alkyle en $C_1$-$C_4$,

A représente un radical $-CH_2-$ ou l'un des radicaux

$-CH_2-CH(R_5)-$, 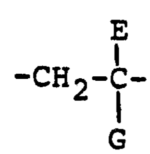 et 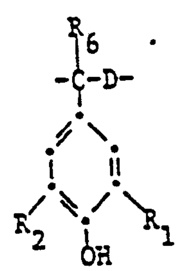

dans lesquels $R_5$ et $R_6$ représentent chacun l'hydrogène ou un méthyle, E représente un radical cyano, $-COCH_3$ ou $-COOCH_3$, G l'hydrogène, un alkyle en $C_1$-$C_{18}$, un allyle, un cyclohexyle, un benzyle ou un radical:

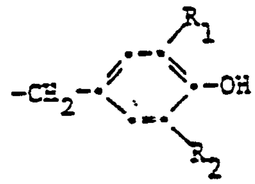

ou encore E et G forment ensemble un radical $-CO-(CH_2)_4-$, et D représente un radical $-(C_rH_{2r})-$ dont l'indice r désigne un nombre de 0 à 6,

et

X représente un radical répondant à l'une ou à l'autre des formules:

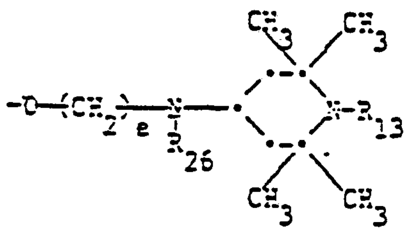

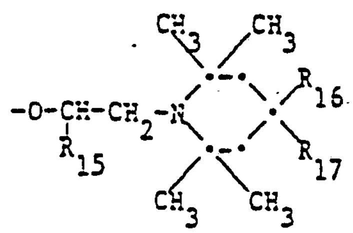

dans lesquelles:

e désigne le nombre 2 ou le nombre 3,

$R_{13}$ représente un radical hydroxy, méthyle, allyle, benzyle, hydroxy-2 éthyle, acétyle ou acryloyle ou un radical $-CON(R_{19})(R_{20})$ dans lequel $R_{19}$ représente un alkyle en $C_1$-$C_8$, un cyclohexyle ou un phényle et $R_{20}$ l'hydrogène ou un alkyle en $C_1$-$C_{12}$,

$R_{26}$ représente l'hydrogène, un alkyle en $C_1$-$C_8$ ou l'un des radicaux $-COR_{27}$, $COOR_{28}$, $-CON(R_{29})(R_{30})$, $-CH_2-CH(R_{31})-OR_{32}$ et $-SO_2R_{33}$ dans lesquels:

$R_{27}$ représente un alkyle en $C_1$-$C_{12}$, un alcényle en $C_2$-$C_4$, un cyclohexyle, un benzyle, un phényle, un (ditert-butyl-3,5 hydroxy-4 phényl)-2 éthyle ou un noyau hétérocyclique,

$R_{28}$ représente un alkyle en $C_1$-$C_8$, un allyle ou un cyclohexyle,

$R_{29}$ représente un alkyle en $C_1$-$C_{12}$, un cyclohexyle ou un phényle,

$R_{30}$ représente l'hydrogène ou un alkyle en $C_1$-$C_{12}$, ou encore

$R_{29}$ et $R_{30}$ forment ensemble, et avec l'atome d'azote auquel ils sont liés, un radical hétérocyclique hexagonal,

$R_{31}$ représente l'hydrogène, un méthyle ou un phényle,

$R_{32}$ représente l'hydrogène, un alkyle en $C_1$-$C_4$, un radical $-COR_{21}$ ou un radical $-CON(R_{19})(R_{20})$ et

$R_{33}$ représente un alkyle en $C_1$-$C_{12}$, un phényle ou un alkylphényle en $C_7$-$C_{10}$,

$R_{15}$ représente l'hydrogène, un méthyle ou un phényle,

$R_{16}$ représente l'hydrogène ou l'un des radicaux $-OR_{34}$, $-OCOR_{35}$ et $-N(R_{36})-COR_{35}$ dans lesquels:

$R_{34}$ représente l'hydrogène, un alkyle en $C_1$-$C_8$, un allyle, un benzyle ou un phényle,

$R_{35}$ représente l'hydrogène, un alkyle en $C_1$-$C_8$, un allyle, un benzyle ou un phényle et

$R_{36}$ représente l'hydrogène, un alkyle en $C_1$-$C_4$, un cyclohexyle ou un benzyle,

et

$R_{17}$ représente l'hydrogène, un radical cyano, $-COOR_{18}$, $-CONH_2$ ou $-CON(R_{19})(R_{20})$ ou encore un radical $-CH_2NHR_{37}$ dans lequel:

$R_{37}$ représente un radical $-COR_{21}$, $-COOR_{18}$, $-CON(R_{19})(R_{20})$ ou $-CH_2-CH(R_{22})-OR_{23}$,

les radicaux et symboles mentionnés à plusieurs reprises dans cette revendication ayant toujours la signification qui leur a été donnée la première fois dans cette même revendication.

3. Matière pour enregistrement photographique en couleurs selon la revendication 2, matière caractérisée en ce qu'elle contient, comme stabilisant, au moins un composé polyalkyl-pipéridinique de formule V dans lequel:

n désigne le nombre 0 ou le nombre 1,
$R_1$ représente l'hydrogène, un méthyle ou un tert-butyle,
$R_2$ représente un méthyle ou un tert-butyle,
A représente un radical $-CH_2-$ ou $-CH_2-CH_2-$ et
X représente un radical de formule:

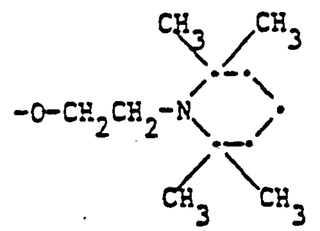

ou un radical répondant à la formule:

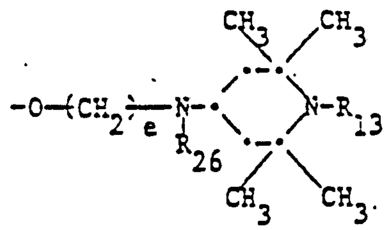

dans laquelle:
e représente le nombre 2 ou le nombre 3,
$R_{13}$ représente un méthyle, un allyle, un benzyle, un hydroxy-2 éthyle, un acétyle ou un acryloyle et
$R_{26}$ représente l'hydrogène, un alkyle en $C_1$-$C_8$ ou l'un des radicaux $-COR_{27}$ et $-SO_2R_3$ dans lesquels:
$R_{27}$ représente un alkyle en $C_1$-$C_{12}$ ou un alcényle en $C_2$-$C_4$ et
$R_{33}$ représente un alkyle en $C_1$-$C_4$, un phényle ou un p-tolyle.

4. Matière pour enregistrement photographique en couleurs selon la revendication 1, matière caractérisée en ce qu'elle contient, comme stabilisant, au moins un composé polyalkyl-pipéridinique de formule I dans lequel A représente l'un des radicaux $-CH_2$, $-CH_2-CH(R_5)-$,

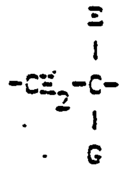

et

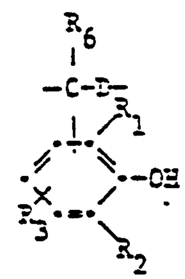

5. Matière pour enregistrement photographique en couleurs selon la revendication 1, matière caractérisée en ce qu'elle contient, comme stabilisant, au moins un composé polyalkyl-pipéridinique répondant à la formule VI:

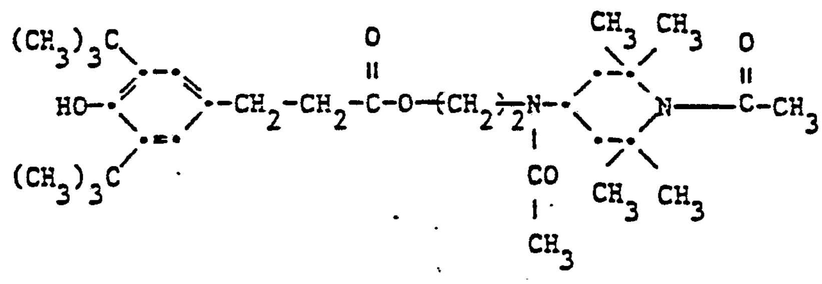

VI.

6. Matière pour enregistrement photographique en couleurs selon la revendication 1, matière caractérisée en ce qu'elle contient, comme stabilisant, au moins un composé polyalkyl-pipéridinique répondant à la formule VII:

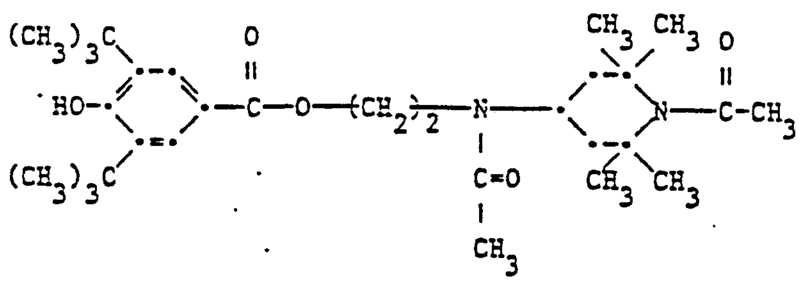

VII.

7. Matière pour enregistrement photographique en couleurs selon la revendication 1, matière caractérisée en ce qu'elle contient, comme stabilisant, au moins un composé polyalkyl-pipéridinique répondant à la formule VIII:

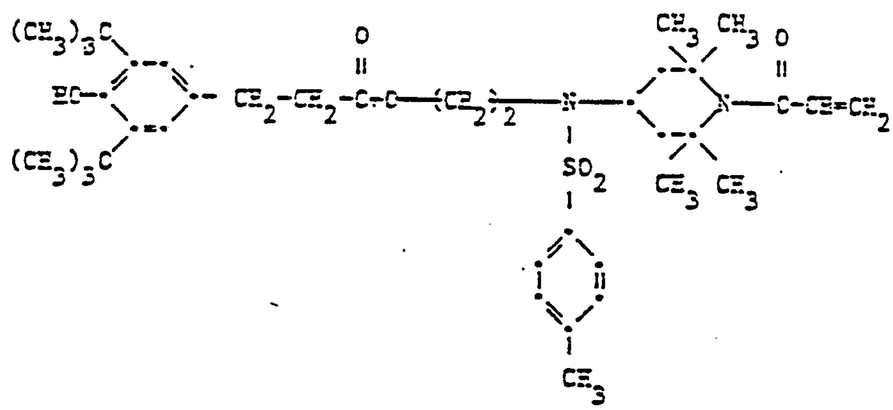

(VIII)

8. Matière pour enregistrement photographique en couleurs selon la revendication 1, matière caractérisée en ce qu'elle contient, comme stabilisant, au moins un composé polyalkyl-pipéridinique répondant à la formule IX:

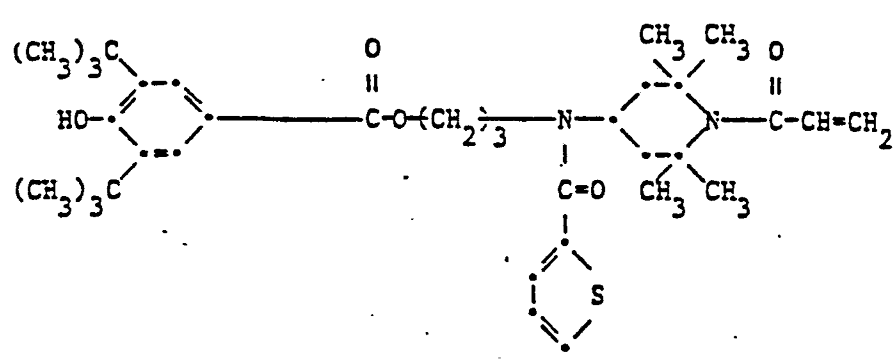

(IX).

9. Matière pour enregistrement photographique en couleurs selon la revendication 1, matière caractérisée en ce qu'elle contient, comme stabilisant, au moins un composé polyalkyl-pipéridinique

répondant à la formule X:

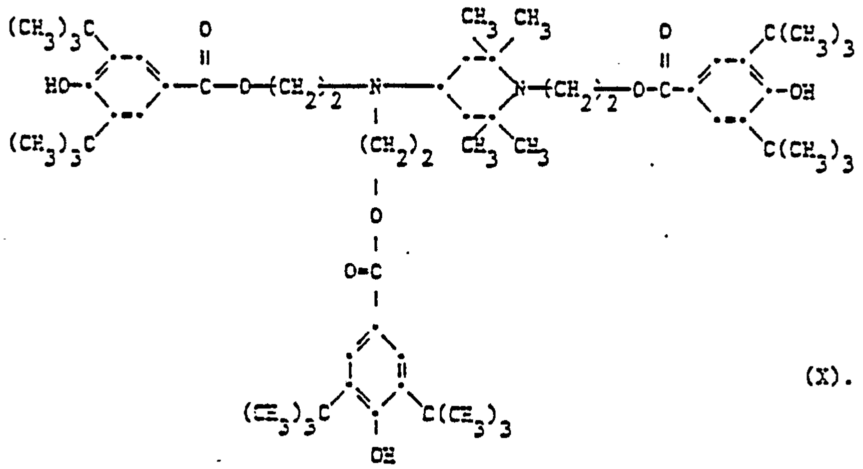

(X).

10. Matière pour enregistrement photographique en couleurs selon la revendication 1, matière caractérisée en ce qu'elle contient les stabilisants de formule I en association avec des copulants turquoise, pourpre et jaune.

11. Matière pour enregistrement photographique en couleurs selon la revendication 1, matière caractérisée en ce qu'elle contient les stabilisants de formule I en association avec des absorbeurs de rayons ultra-violets.

12. Matière pour enregistrement photographique en couleurs selon la revendication 11, caractérisée en que les absorbeurs de rayons ultra-violets sont des composés du type de la benzophénone, de l'acrylonitrile, de la thiazolidone, du benzotriazole, de l'oxazole, du thiazole ou de l'imidazole.

13. Matière pour enregistrement photographique en couleurs selon la revendication 1, matière caractérisée en ce qu'elle contient, dans la même couche, les stabilisants de formule I en association avec des copulants turquoise, pourpre et jaune et avec des absorbeurs de rayons ultraviolets.

14. Matière pour enregistrement photographique en couleurs selon la revendication 1, matière caracterisée en ce qu'elle contient de 1 à 2000 mg du stabilisant de formule I par mètre carré de la couche dans laquelle ledit stabilisant est incorporé.

15. Procédé pour réaliser des images photographiques en couleurs, selon lequel on expose à la lumière conformément à une image une matière pour enregistrement photographique en couleurs selon la revendication 1 et on développe les couleurs.